(19) Europäisches Patentamt
European Patent Office
Office européen des brevets

(11) EP 3 045 541 A1

(12) **EUROPEAN PATENT APPLICATION**
published in accordance with Art. 153(4) EPC

(43) Date of publication:
**20.07.2016 Bulletin 2016/29**

(21) Application number: **14843658.7**

(22) Date of filing: **11.09.2014**

(51) Int Cl.:
***C12Q 1/68*** *(2006.01)* ***C12N 15/09*** *(2006.01)*

(86) International application number:
**PCT/JP2014/074145**

(87) International publication number:
**WO 2015/037681 (19.03.2015 Gazette 2015/11)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **11.09.2013 JP 2013188806**

(71) Applicants:
- **Kyoto University**
  **Kyoto-shi, Kyoto 606-8501 (JP)**
- **Wakayama Medical University**
  **Wakayama 641-8509 (JP)**
- **Ito, Koichi**
  **Tokyo 150-8308 (JP)**
- **Kuma Hospital, Shinkokai Medical Corporation**
  **Kobe-shi, Hyogo 650-0011 (JP)**
- **TOYO KOHAN CO., LTD.**
  **Chiyoda-ku**
  **Tokyo**
  **102-8447 (JP)**

(72) Inventors:
- **MATSUDA, Fumihiko**
  **Kyoto-shi**
  **Kyoto 606-8501 (JP)**
- **TERAO, Chikashi**
  **Kyoto-shi**
  **Kyoto 606-8501 (JP)**
- **AKAMIZU, Takashi**
  **Wakayama-shi**
  **Wakayama 641-8509 (JP)**
- **YOSHIMURA, Hiroshi**
  **Inzai-shi**
  **Chiba 270-1323 (JP)**
- **MIYAUCHI, Akira**
  **Kobe-shi**
  **Hyogo 650-0013 (JP)**

(74) Representative: **Hoffmann Eitle**
**Patent- und Rechtsanwälte PartmbB**
**Arabellastraße 30**
**81925 München (DE)**

# (54) TEST METHOD FOR EVALUATING THE RISK OF ANTI-THYROID DRUG-INDUCED AGRANULOCYTOSIS, AND EVALUATION KIT

(57) The present invention provides a test method and an evaluation kit for determining the risk of antithyroid drug-induced agranulocytosis. More particularly, it provides a test method for determining the risk of antithyroid drug-induced agranulocytosis, including testing susceptibility polymorphism to antithyroid drug-induced agranulocytosis, and determining the risk of antithyroid drug-induced agranulocytosis, and an evaluation kit for the risk of antithyroid drug-induced agranulocytosis, containing a polynucleotide capable of detecting susceptibility polymorphism to antithyroid drug-induced agranulocytosis.

EP 3 045 541 A1

SEQUENCE LISTING

<110> Kyoto University
WAKAYAMA MEDICAL UNIVERSITY
ITO, Koichi
KUMA HOSPITAL, SHINKOKAI MEDICAL CORPORATION

<120> Method of determining risk of antithyroid drug-induced agranulocytosis and kit therefor

<130> 092224

<150> JP2013-188806
<151> 2013-09-11

<160> 5

<170> PatentIn version 3.5

<210> 1
<211> 1001
<212> DNA
<213> Homo sapiens

<400> 1
aatctttcat atggtatttt caccacacct tttgtatgtt tagatacata aacagttagc 60

attgtgttac aattaccaat agtattcaat acagtcacat gctgtacagg tttgtcgcct 120

aggggtaata gggtgtacca tatagcctaa atgtatagta ggctataaca tctagtttgc 180

gtaaggacac tctgtgatgt tcacacaaag atgaaatcac ctaatgacac atttctcaga 240

gcttgtccct ttagctaagt gatgcctgac ttcagttttg ccccatttct agagcatagt 300

cctccatgac tttcaatgaa aaacccgata gctttcatct cctcaatcct gaagagctga 360

aggagattta ggctgaactt aaagaaattt tcagcttagc tcattagtct tctactccat 420

acatcttcaa catttaacaa gtgttttgaa aaagacacct acaaagtgct tgaagtcatc 480

aactctcaaa tcttgtcatt kcagcaccac gtcaaatgac aaaacacttg ctattttctt 540

agtccactgg aggagcctat tgtcagaggc caaacctgga ttattagctc caaagaagca 600

ctcagatcag taagtgtcct caggtgataa gtggttgttg ctacttggca tcaattcacc 660

agttcttctg aaacttacgt ctgttttgtt ttagggccct tatcaatggt aggtctttgt 720

ttcctcaaca ccactggaca gtgaaagatt ttgcactgcc tttcagaagt tgacacttta 780

gtttttttgtt ttaccttcta ccgtagcatc agaagttaac caacgtgttt tgaagaaacc 840

agagtgtttg agatgcctca gttttctagt tacatcacac tggccccata attgctgctg 900

atttctttct tacagcagaa aactgtagga aaattgtagc agaaaacttt tctacagcag 960

aaaacggtag cagaaaaatg gcactaaaac gcagcgtaca c 1001

<210> 2
<211> 401
<212> DNA

<213> Homo sapiens

<400> 2
cacggttccc aaggctgctg caggggtcaa aggggacccc tgatcagtat tctagggact 60
gtcttcccct ccatttcctc agagacgtca ttccttaatt gtctagagag aagagggggc 120
cctcagagga aactcaggaa aactcatgcc attctccatt caacggaggg cgacattcta 180
gcgctgatcc cattttcctc ytcttctcgt gggaggccat ccccggcgac ctataggaga 240
tggggaaggc tccccactgc ccctggtacc agcgcgctcc agcttgtcct tcccgttctc 300
caggtatctg cggagccact ccacgcactc gccctccagg taggctctcc gctgctccgc 360
ctcacgggcc gcctcccact tgcgctgggt gatctgagcc g 401

<210> 3
<211> 1001
<212> DNA
<213> Homo sapiens

<400> 3
aacctgactg tgggggaaat gattctgact gtctcttatt gtaaacttac caggcagcga 60
ctacactaag aacaaaaaca ttggctcagg aaaggcaagg tgaggccaca gagcacagaa 120
caaagcccaa aaaacagccc actgggtact atgaccctcg ggggctggaa aaagtaacac 180
ctggacatgg gatgaaaaca gggaccacag ctgccctgac agagggctgg tccccactcc 240
ccaaatagcc cagggacatc tgcttatcaa ctggtccata ttatctgcaa ggaaacacag 300
ggagacaggg gccatatggt gggaacccag aaaaagcacg gtctcgaggg acccagagga 360
cgtgacaccc ctgagacagc tcccagatga ggcatatggg gagctgcaaa gtggacagag 420
gatggccatg tgcactcagg actctccctg ttacaagggg acctcaaagg ggctgtacac 480
atgggggccc tcattctggg yctcgtgggt ctttttcttg atgtcctcct gatggctgga 540
gaaacagggg agggggatgc agagaggaag ggactagagg caccacctct ccttggattc 600
ctctccagtt tctagccctc cctagatcac atctgccttt actatttgct ccctctgaga 660
tagtgatcat ccaggccctc agcaatcagc acgcaattcc caactcaccc acctggatgc 720
gacctggtaa gcctgagaga cagagaccgg gatggggaca aagcaggcac cacggccctc 780
cctgctgccc actcctcacc tgcagcagga ggaggccaca gctggatgtt cgagggcctt 840
ggcccagccc tggcttgggc aggacttaag ggtgtaaaaa ataacctaca tgtgatggtt 900
cattttcaat tctatgtgcc ttagtatagg tttaagcagg ccacatggtc ataaagagat 960
aaagaaggaa aatgtactaa gccaccatcc cccctacttc t 1001

<210> 4
<211> 1001
<212> DNA
<213> Homo sapiens

<400> 4
gaagttgagg caggaaaatg gcgtgaaccc aagaggcaga gcttgcagta agccgagatc 60

gcaccactgc actccagcct gggcgacaga gcgagactcc atctcaaaaa aaagaaaaaa 120

aaaaaaaaaa aaaaaggaaa accattttaa tagactttta tttttagagc tgttttaagc 180

taacagaaaa attgcagaaa ttgtatacag agctccccca cccccagttt ctacaatgct 240

taacatcctg tattaatgtg gtacacttgt tacaattgat gaaccaatac taataattat 300

tattaactaa aattcatagt tatacgaggg ttcactctgt attacacagt tatatgggtt 360

ctgacaaata cataatatca tatatccacc attacaggat taaacaaaat agcttcactg 420

atctaaaaat gacccaggct ccatctactc atccttcctt cctccctctg aaccattggc 480

attctctgag ctatttacta ktgttttgcc tttttcagaa tgtcacatac ttgtaatcat 540

acagcataga gctttttcag atgagattct tttgcttagc catatgcata caggtttcct 600

gcgtatattg tcatagcttg atagcttatt tttctttaat gttaaataat actccattgt 660

ataaatgtac tatggtttat ttacccatta atctattgaa ggacatcttg gttgcttcta 720

atttttggca attatgaata aagctgctat aaacatccat gaacagatgt ttgtgcagac 780

acaagttttc cactttggat aaatacatag aagggcagtt gctggatcat atggtaagag 840

tatgtttagc tttgtaagaa acaactagaa tatcttccaa aatggctgta tcattttgca 900

ttcctaccag caacgaatga gagtccctgt tgttctatat ccttgccagc atttggtatt 960

ctggggtttg ggatttcagc aagaaagcca ttttaatatt t 1001

<210> 5
<211> 1001
<212> DNA
<213> Homo sapiens

<400> 5
gagtcttact ctgttgccca ggctggagtg tagtggcatg atcttggctc actgcaacct 60

ccgccttccg ggttcaagcg atttccggct aatttttgta tttttagtag ggacggggtt 120

tcaccatgtt ggccaggctg gtctcaaact cttgacctca agtgatccgc ccgcttcagc 180

ctcccaaagt gctaggatta caggcatgag ccactgcgcc cgcctcgttt tgctattctt 240

caacttcgca tgtttggatt gctacagctt gaggtcttta gtgtctggtt tccttcgctc 300

ccttccacgg ttctaagatt gttttctgca tcctccgtac ccctccctcg gtgtcttcct 360

gctgggaact gctctttctg tctgccacgt ggtggccaca agggggcagc agaggctgag 420

gaaaatctgg tgagaccagg ttgggggcct tttcccgggc ccacgagtta ctcccccccg 480

cccacggagc accttctgtc ygggcgcctc ccaggccgct gctgcttctt ggttctgctt 540

ccttttctga gacccgctgc tttagagaag atttctggag cagagatttg gagcaaggat 600

```
ctccaaactc ttttgatcac acactccatt cagtaatata ttttgaacat gcagctaaca     660
aacatatgca aacataatgc aggttgaaag aacaaacgca aatcacatta aaagggcata     720
agattgaaga ttttataata gttctaatat gttctctgcc ttctttcctg tctctccaga     780
tccctggagg acccctaggg ctggtgcccc taatctggtg gccccctgtt tagagctcaa     840
ctgagctttg atgtagacct ggccccctgtt agggtttggc cacacgacct gtgaccctgg    900
gcaggtttcc tcagatcatc gaggctctgt ctctcagctg taaagtgagg acagtaagga     960
ccatctcatg gtattaagct aaaacattca cggaaataaa t                        1001
```

## Description

Technical Field

The present invention relates to a test method and an evaluation kit for determining the risk of antithyroid drug-induced agranulocytosis. More particularly, it relates to a test method for determining the risk of antithyroid drug-induced agranulocytosis, comprising testing susceptibility polymorphism to antithyroid drug-induced agranulocytosis, and determining the risk of antithyroid drug-induced agranulocytosis, and an evaluation kit for the risk of antithyroid drug-induced agranulocytosis, comprising a polynucleotide capable of detecting susceptibility polymorphism to antithyroid drug-induced agranulocytosis.

Background Art

Hyperthyroidism is a disease wherein thyroid hormone is secreted in large amounts from the thyroid gland, and mostly caused by Graves' disease. Graves' disease is an autoimmune disease caused by unlimited stimulation of thyroid gland by an autoantibody to a thyroid-stimulating hormone receptor (TSH Receptor Antibody: TRAb), and characteristically often found in female.

At present, the treatment methods of hyperthyroidism are largely divided into three: drug therapy, radioisotope therapy and operative treatment. Generally, the treatment is started with a drug therapy using an antithyroid drug. Antithyroid drug is a medicament that suppresses synthesis of thyroid hormone, and the blood concentration of thyroid hormone generally returns to normal in several months from the start of medication. However, in the case of Graves' disease, for example, even if the blood concentration of thyroid hormone becomes normal, medication of an antithyroid drug needs to be continued as long as the causative TRAb is positive. Consequently, the treatment continues for a long term in many cases.

As one of the serious side effects of antithyroid drugs, agranulocytosis is known. Agranulocytosis refers to a condition associated with a marked decrease (not more than 500/μL or not more than 100, 200) in the granulocytes, showing a non-specific sterilizing action, from among the leukocytes responsible for the immune response against foreign antigens such as bacterium, virus and the like. About 70% of the agranulocytosis patients are assumed to develop agranulocytosis due to medicaments such as antithyroid drugs (e.g., methimazole) and the like.

The onset frequency of antithyroid drug-induced agranulocytosis is about one in 300 people and is not very high. However, once affected with this disease, the number of granulocytes in blood markedly decreases, which in turn creates an immunocompromised state where lethal pathology could occur due to serious infections. In fact, several fatal cases have been reported annually at present. When an antithyroid drug is administered to patients, therefore, in view of the serious side effects caused thereby, medical doctors should monitor periodically and in detail the leukocyte number and granulocyte number of the patients as long as the medicament is administered, even though the onset frequency of agranulocytosis is low. This places an excessive burden on medical doctors and patients particularly when the treatment is necessary for a long term.

Generally, moreover, subjective symptoms of agranulocytosis scarcely appear at the time point when granulocytes start to decrease. In many cases, therefore, infections have already progressed when the disease is diagnosed, in which case administration of antithyroid drug is discontinued, the treatment of hyperthyroidism is suspended, and an appropriate treatment of infection is performed. Consequently, the patients suffer from dual hardships.

For the above reasons, the development of a test method capable of predicting the onset risk of antithyroid drug-induced agranulocytosis is desired, and the possibility of elderly people, female, people with kidney hypofunction and the like being high-risk groups has been suggested. As mentioned above, however, since the onset frequency of antithyroid drug-induced agranulocytosis is low and case accumulation is extremely difficult, the results sufficiently reliable for establishing the possibility have not been obtained.

Human leukocyte antigen (HLA), a major histocompatibility complex (MHC) of human, is a huge composite gene region of about 3.6 Mb and is present in the 6p21.3 region of the short arm of human chromosome 6. This region contains genetic information of many proteins involved in immune reactions.

HLA region is mainly classified into (1) class I region governing HLA-A, B, C antigen systems and the like, (2) class III region governing complement components and the like, and (3) class II region governing HLA-DP, DQ, DR antigen systems and the like, from the telomere side to the centromere side of the chromosome.

HLA gene shows the highest polymorphism among functional genes, and the presence of a disease susceptibility gene in the HLA region can be tested by, for example, comparing HLA allele frequency between patient population and healthy control population. When a significant association is found between a particular HLA allele and a disease by association study, the HLA allele itself may determine the disease susceptibility, or a different gene in the HLA region in linkage disequilibrium with the HLA allele may determine the disease susceptibility.

Up to the present, it has been clarified by case-control study that particular HLA allele significantly increases or decreases in a patient population of a particular disease, and there are many reports relating to immunity-associated diseases among others.

Genetic analyses based on the comparison of HLA allele frequency are also ongoing for antithyroid drug-induced agranulocytosis, and it has been reported that HLA-DRB1*08:03:02 is associated with an antithyroid drug, methimazole-induced agranulocytosis, in Japanese people (non-patent document 1). However, the number of patients samples used for the study was only 24, and the target gene was limited to HLA class II gene. Therefore, it is not clear whether HLA-DRB1*08:03:02 is a disease susceptibility gene to methimazole-induced agranulocytosis, and reverification of the results is necessary. In fact, clinical application has not been implemented.

In recent years, genome wide association study (GWAS) has been actively used as a method of analyzing a causative gene of a disease. GWAS is a method of identifying a gene associated with a particular disease by comprehensively analyzing the SNPs that the patients affected with the disease have in the whole genome, by using single nucleotide polymorphism (SNP) present in the human genome as an index.

However, GWAS has not been performed for drug-induced agranulocytosis till date, and a useful clinical test method capable of determining the risk of antithyroid drug-induced agranulocytosis has not been developed.

[Document List]

[non-patent document]

non-patent document 1: Tamai H et al., Ann Intern Med., 124(5):490-494 (1996)

SUMMARY OF THE INVENTION

Problems to be Solved by the Invention

An object of the present invention is to provide a test method and an evaluation kit for conveniently and highly accurately determining the risk of antithyroid drug-induced agranulocytosis.

Means of Solving the Problems

In view of the above-mentioned object, the present inventors collected, in cooperation with two facilities of representative special hospitals of thyroid gland in Japan, 115 samples of antithyroid drug-induced agranulocytosis patients with strict and detailed clinical information, and searched for a genetic factor related to antithyroid drug-induced agranulocytosis by a case-control study of GWAS using human SNP array.

To be specific, 115 patient samples were typed using SNPs present at 2,635,435 sites on the genome as markers. As a control group, typing information of DNA samples (1,798 samples) of general Japanese people, which were collected from the community-based genome cohort, was used, and case-control study including comparison of the genotype frequency of each SNP was performed.

As a result, 191 SNPs showing extremely strong association with antithyroid drug-induced agranulocytosis were identified in the human HLA region on the short arm of human chromosome 6 at 6p21.3. They were largely classified into total 4 regions including 2 regions in Class I region (upstream side of HLA-A region and around HLA-B region) 2 regions in Class II region (2 regions around HLA-DR region). Of these, the strongest association was obtained around HLA-DR region (most strongly associated polymorphism: rs6457580), followed by around HLA-B region (most strongly associated polymorphism: rs41560220). Many of SNPs showing significant difference in each region are in strong linkage disequilibrium. Since mutual linkage disequilibrium among 4 regions was not found, they were found to be independent.

Since all the polymorphism markers obtained above are present in the HLA gene region, antithyroid drug-induced agranulocytosis may be related to HLA genes per se. Therefore, the targets were narrowed down to around HLA-DR region and HLA-B region that showed extremely strong association, and genotyping of HLA-B, HLA-C, HLA-DRB1, HLA-DPB1 and HLA-DQB1 gene was performed using patient samples. As a result, HLA alleles of HLA-B*39:01 and HLA-B*38:02, as well as HLA-DRB1*08:03, HLA-DRB1*14:03 and HLA-DRB1*08:02 were correlated with antithyroid drug-induced agranulocytosis. In addition, HLA-B*39:01 and HLA-B*38:02 were in linkage disequilibrium with rs41560220, and HLA-DRB1*08:03 and HLA-DRB1*08:02 were in linkage disequilibrium with rs6457580.

Since plural alleles were correlated to agranulocytosis in both of HLA-B and HLA-DRB1 genes, the possible presence of an important amino acid residue in these susceptibility HLA alleles was considered. Thus, set up multiple logistic regression analysis was performed using the alignment of HLA amino acid sequences. As a result, the phenylalanine residue (B-Phe116) at position 116 of HLA-B protein and the leucine residue (DRB1-Leu74) at position 74 of HLA-DRB1 protein were strongly correlated with antithyroid drug-induced agranulocytosis. In addition, the absence of alanine residue at position 158 of HLA-B protein was found to be in linkage disequilibrium ($r^2$=0.92) with the presence of B-Phe116.

The present inventors have conducted further studies based on these findings and confirmed that the risk of antithyroid drug-induced agranulocytosis can be evaluated by testing these polymorphisms, which resulted in the completion of the present invention.

Accordingly, the present invention relates to the following.

[1] A test method for determining the risk of antithyroid drug-induced agranulocytosis, comprising

(1) a step of using a sample derived from a test subject and testing polymorphism present in the HLA region, which is at least one selected from the group consisting of

A) polymorphism at the 501st nucleotide in the nucleotide sequence shown in SEQ ID NO: 1 (G>T*),
B) polymorphism at the 201st nucleotide in the nucleotide sequence shown in SEQ ID NO: 2 (C>T*),
C) polymorphism at the 501st nucleotide in the nucleotide sequence shown in SEQ ID NO: 3 (C>T*),
D) polymorphism at the 501st nucleotide in the nucleotide sequence shown in SEQ ID NO: 4 (T*>G),
E) polymorphism at the 501st nucleotide in the nucleotide sequence shown in SEQ ID NO: 5 (C>T*),
wherein parentheses show reference allele>variant allele, * is risk allele, G, A, T and C are guanine, adenine, thymine and cytosine, respectively, and
F) polymorphism in linkage disequilibrium with the polymorphism of any of the above-mentioned A) - E), the linkage disequilibrium showing a linkage disequilibrium coefficient D' of not less than 0.8, and

(2) a step of determining the risk of antithyroid drug-induced agranulocytosis based on the test results of (1);

[2] the test method of the above-mentioned [1], comprising a step of testing the polymorphism of the above-mentioned A) or polymorphism in linkage disequilibrium with said polymorphism at a linkage disequilibrium coefficient D' of not less than 0.8, and/or the polymorphism of B) or polymorphism in linkage disequilibrium with said polymorphism at a linkage disequilibrium coefficient D' of not less than 0.8;
[3] the test method of the above-mentioned [2], wherein the polymorphism in linkage disequilibrium with the polymorphism of the above-mentioned A) at a linkage disequilibrium coefficient D' of not less than 0.8 is polymorphism at a position encoding the amino acid at position 74 of HLA-DRB1*08:03 or HLA-DRB1*08:02, and the polymorphism in linkage disequilibrium with the polymorphism of the above-mentioned B) at a linkage disequilibrium coefficient D' of not less than 0.8 is polymorphism at a position encoding the amino acid at position 116 or position 158 of HLA-B*39:01 or HLA-B*38:02;
[4] the test method of any of the above-mentioned [1] - [3], wherein the sample derived from the test subject contains genomic DNA;
[5] the test method of any of the above-mentioned [1] - [4], wherein the test subject is an eastern Asian;
[6] a kit for determination of the risk of antithyroid drug-induced agranulocytosis, comprising a polynucleotide capable of detecting a risk allele in
polymorphism present in the HLA region, which is at least one selected from the group consisting of

A) polymorphism at the 501st nucleotide in the nucleotide sequence shown in SEQ ID NO: 1 (G>T*),
B) polymorphism at the 201st nucleotide in the nucleotide sequence shown in SEQ ID NO: 2 (C>T*),
C) polymorphism at the 501st nucleotide in the nucleotide sequence shown in SEQ ID NO: 3 (C>T*),
D) polymorphism at the 501st nucleotide in the nucleotide sequence shown in SEQ ID NO: 4 (T*>G),
E) polymorphism at the 501st nucleotide in the nucleotide sequence shown in SEQ ID NO: 5 (C>T*),
wherein parentheses show reference allele>variant allele, * is risk allele, G, A, T and C are guanine, adenine, thymine and cytosine, respectively, and
F) polymorphism in linkage disequilibrium with the polymorphism of any of the above-mentioned A) - E), the linkage
disequilibrium showing a linkage disequilibrium coefficient D' of not less than 0.8;

[7] the kit of the above-mentioned [6], comprising a polynucleotide capable of detecting a risk allele of the polymorphism of the above-mentioned A) or polymorphism in linkage disequilibrium with said polymorphism at a linkage disequilibrium coefficient D' of not less than 0.8 and/or the polymorphism of B) or polymorphism in linkage disequilibrium with said polymorphism at a linkage disequilibrium coefficient D' of not less than 0.8;
[8] the kit of the above-mentioned [7], wherein the polymorphism in linkage disequilibrium with the polymorphism of the above-mentioned A) at a linkage disequilibrium coefficient D' of not less than 0.8 is polymorphism at a position encoding the amino acid at position 74 of HLA-DRB1*08:03 or HLA-DRB1*08:02, and the polymorphism in linkage disequilibrium with the polymorphism of the above-mentioned B) at a linkage disequilibrium coefficient D' of not less than 0.8 is polymorphism at a position encoding the amino acid at position 116 or position 158 of HLA-B*39:01 or HLA-B*38:02;
[9] the kit of the above-mentioned [6] - [8], further comprising a polynucleotide capable of detecting a non-risk allele;
[10] the kit of any of the above-mentioned [6] - [9], wherein the above-mentioned polynucleotide capable of detecting a risk allele is a probe capable of hybridizing with a fragment of 10 - 200 continuous nucleotide sequence containing said allele or a complementary chain sequence thereof under stringent conditions, and/or a primer capable of amplifying said fragment;
[11] the kit of any of the above-mentioned [6] - [10], which is used for determining the risk of antithyroid drug-induced agranulocytosis in eastern Asians;
[12] a test method for determining the risk of antithyroid drug-induced agranulocytosis, comprising

(1) a step of using a sample derived from a test subject and testing the following (a) and/or (b):

(a) whether the amino acid at position 74 of HLA-DRB1 protein is Leu
(b) whether the amino acid at position 116 of HLA-B protein is Phe, or the amino acid at position 158 of HLA-B protein is Ala, and

(2) a step of determining the risk of antithyroid drug-induced agranulocytosis based on the test results of (1);

[13] a kit for determination of the risk of antithyroid drug-induced agranulocytosis, comprising a substance capable of identifying the following (a) and/or (b):

(a) the amino acid at position 74 of HLA-DRB1 protein is Leu
(b) the amino acid at position 116 of HLA-B protein is Phe, or the amino acid at position 158 of HLA-B protein is Ala.

Effect of the Invention

According to the present invention, the risk of antithyroid drug-induced agranulocytosis can be determined conveniently and highly accurately. Therefore, in patients determined to have a high risk of antithyroid drug-induced agranulocytosis, the onset of extremely serious side effects can be avoided by selecting a treatment method other than a drug therapy in treating hyperthyroidism and, in patients determined to have a low risk thereof, invasive tests such as excessive blood sampling and the like can be avoided. As a result, a safe, secure and accurate treatment of hyperthyroidism becomes possible.

Brief Description of the Drawings

Fig. 1 is a Manhattan plot showing association of antithyroid drug-induced agranulocytosis with SNP from among the SNPs present on the chromosome. P value ($-\log_{10}(P)$) of each SNP obtained by GWAS is shown on the vertical axis and the position on the chromosome is shown on the horizontal axis.
Fig. 2 is an enlarged view of the results of HLA region in Fig. 1.
Fig. 3 is a linkage disequilibrium (LD) map of 6p21.3 region on the short arm of human chromosome 6.
Fig. 4 is a linkage disequilibrium (LD) map of 6p21.3 region on the short arm of human chromosome 6.
Fig. 5 is a linkage disequilibrium (LD) map of 6p21.3 region on the short arm of human chromosome 6.
Fig. 6 shows agranulocytosis odds ratios relative to the number of risk alleles in 4 SNP markers (rs6457580, rs41560220, rs1736959 and rs3135387).
Fig. 7 shows association of agranulocytosis and the amino acids at position 74 and position 116 of HLA-DRB1 and HLA-B proteins.

Description of Embodiments

In one embodiment, the present invention provides a test method for determining the risk of antithyroid drug-induced agranulocytosis, comprising

(1) a step of using a sample derived from a test subject and testing polymorphism present in the HLA region, which is at least one selected from the group consisting of

A) polymorphism at the 501st nucleotide in the nucleotide sequence shown in SEQ ID NO: 1 (G>T*),
B) polymorphism at the 201st nucleotide in the nucleotide sequence shown in SEQ ID NO: 2 (C>T*),
C) polymorphism at the 501st nucleotide in the nucleotide sequence shown in SEQ ID NO: 3 (C>T*),
D) polymorphism at the 501st nucleotide in the nucleotide sequence shown in SEQ ID NO: 4 (T*>G),
E) polymorphism at the 501st nucleotide in the nucleotide sequence shown in SEQ ID NO: 5 (C>T*),
wherein parentheses show reference allele>variant allele, * is risk allele, G, A, T and C are guanine, adenine, thymine and cytosine, respectively, and
F) polymorphism in linkage disequilibrium with the polymorphism of any of the above-mentioned A) - E), the linkage disequilibrium showing a linkage disequilibrium coefficient D' of not less than 0.8, and

(2) a step of determining the risk of antithyroid drug-induced agranulocytosis based on the test results of (1) (hereinafter to be also indicated as the test method of the present invention).

In the present specification, the "antithyroid drug" is not particularly limited as long as it can be used for the treatment of hyperthyroidism and can suppress synthesis of thyroid gland-stimulating hormone. Preferably, it means thionamide drugs, for example, carbimazole, methimazole, propylthiouracil and the like, more preferably methimazole or propylthiouracil, most preferably methimazole. As used herein, "hyperthyroidism" means a disease in which thyroid hormone is secreted in large amounts from the thyroid gland, and examples thereof include, but are not limited to, Graves' disease, inflammation due to toxic substance and radiation, and the like.

In the test method of the present invention, the "test subject" to be the measurement target refers to hyperthyroidism patients, preferably Graves' disease patients, and is a human who is or is scheduled to be under medication of an antithyroid drug for the treatment or prophylaxis of the disease. While the race of the test subject is not particularly limited, preferred are eastern Asians, more preferred are Japanese people.

The "sample derived from a test subject" to be the measurement target in the test method of the present invention is preferably a biological sample containing the genomic DNA of the test subject. When the polymorphism to be detected is present in the region located in mRNA, which is other than non-transcribed regions such as promoter and the like, regions removed by RNA splicing such as intron and the like, biological samples containing mRNA and total RNA may be used instead of genomic DNA.

The sample may be, for example, a biological tissue of the test subject, specifically, for example, excreta such as feces, urine, expectoration, saliva and the like, body fluid such as blood and the like, cells of mouth cavity mucosa, skin and the like, body hair and the like may be directly used. Alternatively, genomic DNA isolated from biological tissues of the test subject by a method well known to those of ordinary skill in the art may be used. For example, genomic DNA can be isolated by a phenol extraction method and the like from samples of blood, saliva, skin and the like collected from human. In this event, commercially available genomic DNA extraction kits and apparatuses may be used.

In the present specification, "the risk of antithyroid drug-induced agranulocytosis" means a risk of developing or aggravating agranulocytosis by the administration of an antithyroid drug, and "the high risk of antithyroid drug-induced agranulocytosis" means that the possibility of developing or aggravating agranulocytosis in the future is high. Therefore, the test method of the present invention can be performed, for example, to determine the risk of developing agranulocytosis before administration of an antithyroid drug, or determine the risk of aggravating agranulocytosis during administration of an antithyroid drug.

In the present specification, the "HLA region" means the entire genomic DNA region of about 3.6 Mb from HCP5P15 gene on the telomere side to KIFC1 gene on the centromere side, which is present in the 6p21.3 region of the short arm of human chromosome 6, and does not mean a particular gene segment alone.

In the present specification, the "polymorphism" means changes in one or more nucleotides (substitution, deletion, insertion, translocation, inversion and the like) on genomic DNA and examples thereof include substitution of one nucleotide with other nucleotide (SNP), deletion or insertion of 1 - several dozen nucleotides (DIP), different number of repeats of a sequence consisting of 2 - several dozen nucleotides as one unit (microsatellite polymorphism containing 2 - 4 nucleotides as repeat unit, variable number of tandem repeat (VNTR) containing several - several dozen nucleotides as repeat unit) and the like, with preference given to SNP or DIP.

In the present specification, the "reference allele" of the polymorphism such as SNP and the like shows the same nucleotide or nucleotide sequence as the human genome standard sequence (called RefSeq), and "variant allele" shows allele that emerges as polymorphism but is not the same as RefSeq.

In the present specification, the "risk allele" of the polymorphism such as SNP and the like refers to allele that increases the risk of antithyroid drug-induced agranulocytosis, and "non-risk allele" is an allelic allele of risk allele.

In the present specification, single nucleotide polymorphism (SNP) is shown by the rs number which is a reference SNP ID No. in the SNP database dbSNP[http://www.ncbi.nlm.nih.gov/SNP/] provided by NCBI[http://www.ncbi.nlm.nih.gov/], and the location of the nucleotide is based on NCBI, Genome Reference Consortium Human Build 37 (GRCh37).

In the present invention, the polymorphism of the above-mentioned A) is polymorphism at the 501st nucleotide in the nucleotide sequence shown in SEQ ID NO: 1, and is SNP shown by ID No.: rs6457580 in the SNP database dbSNP provided by NCBI. The polymorphism is SNP wherein 32393141 nucleotide in genomic sequence NC_000006.11 is G>T (reference allele>variant allele, hereinafter the same). The nucleotide sequence shown in SEQ ID NO: 1 shows genomic DNA sequence of human chromosome 6 of each 500 bp before and after the above-mentioned SNP.

As a result of the case-control study by GWAS using human SNP array, allele frequency of T allele was significantly higher in the case group than in the control group. As used herein, "significant" means that the p value in the chi-squared test or Fisher's exact probability test is less than $5.0 \times 10^{-8}$. Therefore, T allele is a risk allele of antithyroid drug-induced agranulocytosis.

In the present invention, the polymorphism of the above-mentioned B) is polymorphism at the 201st nucleotide in the nucleotide sequence shown in SEQ ID NO: 2, and is SNP shown by ID No.: rs41560220 in the SNP database dbSNP provided by NCBI. The polymorphism is SNP wherein 31323875 nucleotide in genomic sequence NC_000006.11 is C>T (reference allele>variant allele, hereinafter the same). The nucleotide sequence shown in SEQ ID NO: 2 shows genomic DNA sequence of human chromosome 6 of 200 bp before and after the above-mentioned SNP.

As a result of the case-control study by GWAS using human SNP array, allele frequency of T allele was significantly higher in the case group than in the control group. Therefore, T allele is a risk allele of antithyroid drug-induced agranulocytosis.

In the present invention, the polymorphism of the above-mentioned C) is polymorphism at the 501st nucleotide in the nucleotide sequence shown in SEQ ID NO: 2, and is SNP shown by ID No.: rs1736959 in the SNP database dbSNP provided by NCBI. The polymorphism is SNP wherein 29782470 nucleotide in genomic sequence NC_000006.11 is C>T (reference allele>variant allele, hereinafter the same). The nucleotide sequence shown in SEQ ID NO: 3 shows genomic DNA sequence of human chromosome 6 of 500 bp before and after the above-mentioned SNP.

As a result of the case-control study by GWAS using human SNP array, allele frequency of T allele was significantly higher in the case group than in the control group. Therefore, T allele is a risk allele of antithyroid drug-induced agranulocytosis.

In the present invention, the polymorphism of the above-mentioned D) is polymorphism at the 501st nucleotide in the nucleotide sequence shown in SEQ ID NO: 4, and is SNP shown by ID No.: rs3135387 in the SNP database dbSNP provided by NCBI. The polymorphism is SNP wherein 32415109 nucleotide in genomic sequence NC_000006.11 is T>G (reference allele>variant allele, hereinafter the same). The nucleotide sequence shown in SEQ ID NO: 4 shows genomic DNA sequence of human chromosome 6 of 500 bp before and after the above-mentioned SNP.

As a result of the case-control study by GWAS using human SNP array, allele frequency of T allele was significantly higher in the case group than in the control group. Therefore, T allele is a risk allele of antithyroid drug-induced agranulocytosis.

In the present invention, the polymorphism of the above-mentioned E) is polymorphism at the 501st nucleotide in the nucleotide sequence shown in SEQ ID NO: 5, and is SNP shown by ID No.: rs17576984 in the SNP database dbSNP provided by NCBI. The polymorphism is SNP wherein 32212985 nucleotide in genomic sequence NC_000006.11 is C>T (reference allele>variant allele, hereinafter the same). The nucleotide sequence shown in SEQ ID NO: 5 shows genomic DNA sequence of human chromosome 6 of 500 bp before and after the above-mentioned SNP.

As a result of the case-control study by GWAS using human SNP array, allele frequency of T allele was significantly higher in the case group than in the control group. Therefore, T allele is a risk allele of antithyroid drug-induced agranulocytosis.

In the present invention, the polymorphism of the above-mentioned F) is polymorphism in linkage disequilibrium with the polymorphism of any of the above-mentioned A) - E), wherein the linkage disequilibrium showing a linkage disequilibrium coefficient D' of not less than 0.8. As used herein, "linkage disequilibrium coefficient D'" is obtained by the following formula

$$D' = (P_{AB}P_{ab} - P_{Ab}P_{aB}) / \mathrm{Min}[(P_{AB}+P_{aB})(P_{aB}+P_{ab}), (P_{AB}+P_{Ab})(P_{Ab}+P_{ab})]$$

wherein, in the two polymorphisms, each allele of the first polymorphism is (A,a), and each allele of the second polymorphism is (B,b), each frequency of 4 haplotypes (AB, Ab, aB, ab) is $P_{AB}$, $P_{Ab}$, $P_{aB}$, $P_{ab}$, respectively, and $\mathrm{Min}[(P_{AB}+P_{aB})(P_{aB}+P_{ab}), (P_{AB}+P_{Ab})(P_{Ab}+P_{ab})]$ means that smaller value of $(P_{AB}+P_{aB})(P_{aB}+P_{ab})$ and $(P_{AB}+P_{Ab})(P_{Ab}+P_{ab})$ is taken.

As the index showing the linkage disequilibrium state, linkage disequilibrium coefficient $r^2$ is sometimes used. The "linkage disequilibrium coefficient $r^2$" is obtained by the following formula

$$r^2=(P_{AB}P_{ab}-P_{Ab}P_{aB})^2/(P_{AB}+P_{aB})(P_{aB}+P_{ab})(P_{AB}+P_{Ab})(P_{Ab}+P_{ab})$$

wherein, in the two polymorphisms, each allele of the first polymorphism is (A,a), and each allele of the second polymorphism is (B,b), each frequency of 4 haplotypes (AB, Ab, aB, ab) is $P_{AB}$, $P_{Ab}$, $P_{aB}$, $P_{ab}$, respectively.

The polymorphism of the above-mentioned F) which is in linkage disequilibrium with the polymorphism of any of the above-mentioned A) - E) can be identified by a method known per se and can also be identified using, for example, HapMap database (http://www.hapmap.org/index.html.ja) and the like. It is also possible to analyze sequences of plural sample DNAs by a sequencer, and search for SNP in a linkage disequilibrium state. For example, the polymorphism of the above-mentioned F) can be identified easily by forming LD block by using Haploview software and according to a method known per se (Figs. 3 - 5).

Examples of the polymorphism of the above-mentioned F) include polymorphism in linkage disequilibrium with the polymorphism of any of the above-mentioned A) - E), wherein the linkage disequilibrium shows a linkage disequilibrium coefficient D' of not less than 0.8, preferably not less than 0.9, more preferably not less than 0.95, further preferably not less than 0.99, most preferably 1 (complete linkage), or a linkage disequilibrium coefficient $r^2$ of not less than 0.6, preferably not less than 0.8, more preferably not less than 0.9, further preferably not less than 0.95, most preferably 1 (complete linkage).

Of the polymorphism of the above-mentioned F), polymorphism in linkage disequilibrium with the polymorphism of the above-mentioned A) at a linkage disequilibrium coefficient D' of 1 (complete linkage) includes, for example, rs28362683, rs10947262, rs4959028, rs6930615, rs732162, rs9501626, rs3135392, rs8084, rs2239806, rs7192, rs3129888, rs7195, rs1051336, rs1041885, rs2213586, rs2213585, rs9268832, rs6903608, rs9268877, rs9268880, rs9268979, rs9269110, rs1964995, rs4713555, rs7744001 and the like.

Of the polymorphism of the above-mentioned F), polymorphism in linkage disequilibrium with the polymorphism of the above-mentioned B) at a linkage disequilibrium coefficient D' of 1 (complete linkage) includes, for example, rs2596487 and the like.

Of the polymorphism of the above-mentioned F), polymorphism in linkage disequilibrium with the polymorphism of the above-mentioned C) at a linkage disequilibrium coefficient D' of 1 (complete linkage) includes, for example, rs1633041, rs1737041, rs1002046, rs1610644, rs1633011, rs1630969, rs1632988, rs1632987, rs1736971, rs1736969, rs1610663, rs1610699, rs11753629, rs1736957, rs1620173, rs1619379, rs2735028, rs1049033 and the like.

Of the polymorphism of the above-mentioned F), polymorphism in linkage disequilibrium with the polymorphism of the above-mentioned D) at a linkage disequilibrium coefficient D' of 1 (complete linkage) includes, for example, rs2395148, rs12524661 and the like.

The test method of the present invention includes a step of testing at least one polymorphism selected from the group consisting of the above-mentioned A) - F) (hereinafter to be also indicated as the polymorphism of the present invention). In one embodiment, a step of testing the polymorphism of the present invention includes a step of detecting the presence or absence of a risk allele of the polymorphism of the present invention. In another embodiment of the present invention, the step of testing the polymorphism of the present invention includes a step of detecting the presence or absence of a risk allele of the polymorphism of the present invention, and a step of detecting the presence or absence of a non-risk allele.

Whether the polymorphism of the present invention contains a risk allele and/or a non-risk allele can be performed by any polymorphism analysis method known in the pertinent field. For example, a method including hybridization according to the method of, for example, Wallace et al. (Proc. Natl. Acad. Sci. USA, 80, 278-282 (1983)) by using genomic DNA extracted from the cells etc. of the test subject as a sample, and a nucleic acid containing a continuous nucleotide sequence of about 15 - about 500 nucleotides containing the nucleotide of any of the above-mentioned polymorphic sites as a probe, while accurately controlling the stringency, and detection of only a sequence completely complementary to the probe, and a method including using a mix probe wherein one of the above-mentioned nucleic acid and the above-mentioned nucleic acid wherein the nucleotide of the polymorphic site is substituted by other nucleotide is labeled and the other is not labeled, performing hybridization by gradually decreasing the reaction temperature from the denaturation temperature, first hybridizing a sequence completely complementary to one of the probes, and preventing cross-reaction with a probe having mismatch and the like can be mentioned. Examples of the label to be used include radioisotope (e.g., $^{32}P$ and the like), enzyme (e.g., β-galactosidase, β-glucosidase, alkaline phosphatase, peroxidase, malic acid dehydrogenase and the like), fluorescent substance (e.g., fluorescamine, fluorescein isothiocyanate, Cy3, Cy5 and the like), luminescence substance (e.g., luminol, luminol derivative, luciferin, lucigenin and the like) and the like.

Preferably, detection of polymorphism can be performed by, for example, various methods described in WO 03/023063, for example, RFLP method, PCR-SSCP method, ASO hybridization, direct sequencing method, ARMS method, denaturing gradient gel electrophoresis, RNase A cleavage method, chemical cleavage method, DOL method, TaqMan PCR method, invader method, MALDI-TOF/MS METHOD, TDI method, molecular beacon method, dynamic allele-specific hybridization method, padlock probe method, UCAN method, nucleic acid hybridization method using DNA chip or DNA microarray, ECA method and the like (WO 03/023063, page 17, line 5 - page 28, line 20. TaqMan PCR method and invader method are explained in detail below as representative methods.

(1) TaqMan PCR method

TaqMan PCR method utilizes PCR using fluorescence labeled allele-specific oligonucleotide (TaqMan probe) and Taq DNA polymerase. As the TaqMan probe, oligonucleotide consisting of a continuous nucleotide sequence of about 15 - about 30 nucleotides containing nucleotide of any of the above-mentioned polymorphic sites is used. In the probe, the 5'-terminal thereof is labeled with a fluorescent dye such as FAM, VIC and the like, and the 3'-terminal is labeled with a quencher such as TAMRA and the like (quenching substance), in which state the fluorescence is not detected since the quencher absorbs the fluorescence energy. A probe is prepared for both alleles, and is preferably labeled with a fluorescent dye having a different fluorescent wavelength (e.g., one of the alleles with FAM, and the other with VIC) for collective detection. The 3'-terminal is phosphorylated to prevent PCR elongation reaction from the TaqMan probe. When PCR is performed using a primer designed to amplify a partial sequence of genome DNA containing a region hybridizing to TaqMan probe and Taq DNA polymerase, the TaqMan probe hybridizes to template DNA, and an elongation reaction from PCR primer occurs simultaneously. When the elongation reaction proceeds, hybridized TaqMan probe is cleaved by the 5' nuclease activity of the Taq DNA polymerase, the fluorescent dye is liberated to be free from the influence of the quencher, and the fluorescence is detected. The fluorescent intensity increases exponentially due to the amplification of the template.

For example, in the detection of the polymorphism of the above-mentioned 1), when allele-specific oligonucleotide containing the nucleotide of the polymorphic site (about 15 - about 30 nucleotide length; 5'-terminal is labeled with FAM for G allele or with VIC for T allele, and the 3'-terminal is labeled with TAMRA) is used as a TaqMan probe, and the genotype of the test subject is GG or TT, then the fluorescent intensity with strong FAM or VIC is observed, and the other fluorescence is hardly observed. On the other hand, when the genotype of the test subject is GT, fluorescence of both FAM and VIC is detected.

(2) Invader method

In the invader method, being different from the TaqMan PCR method, an allele-specific oligonucleotide (allele probe) per se is not labeled, and has a sequence free of complementarity with template DNA (flap) on the 5'-side of a nucleotide of a polymorphic site and a template-specific complementary sequence on the 3'-side. In the invader method, an oligonucleotide having a specific sequence complementary to the 3'-side of the template than the polymorphic site (invader probe; nucleotide corresponding to the polymorphic site at the 5'-terminal of the probe may be any), and a FRET (fluorescence resonance energy transfer) probe wherein the 5'-side has a sequence possibly having a hairpin structure, and a sequence extending from a nucleotide, which forms a pair with the 5'-terminal nucleotide upon formation of the hairpin structure, to the 3'-side is a sequence complementary to the flap of the allele probe are further used. The 5'-terminal of the FRET probe is fluorescence-labeled (e.g., FAM, VIC and the like), and a quencher (e.g., TAMRA and the like) is bound to the vicinity thereof and, in this state (hairpin structure), the fluorescence is not detected.

When a template genomic DNA is reacted with an allele probe and an invader probe, the 3'-terminal of the invader probe invades into the polymorphic site upon complementary binding of these three. When a single strand region of the allele probe (i.e., flap region on 5'-side from the nucleotide of the polymorphic site) is cleaved out using an enzyme (cleavase) that recognizes the structure of the polymorphic site, the flap complementarily binds to the FRET probe, and the polymorphic site of the flap invades into the hairpin structure of the FRET probe. Since the cleavase recognizes and cleaves this structure, the fluorescent dye labeling the terminal of the FRET probe is liberated to be free from the influence of the quencher and the fluorescence is detected. While an allele probe having a nucleotide, which does not match the template, at the polymorphic site is not cleaved by the cleavase, since an allele probe free of cleavage can hybridize to a FRET probe, the fluorescence is detected similarly. However, since the reaction efficiency varies, an allele probe having the nucleotide, which matches the template, at the polymorphic site shows remarkably high fluorescent intensity as compared to an allele probe without matching.

Generally, before reaction of the three kinds of probes and cleavase, template DNA is preferably amplified by PCR using a primer capable of amplifying the region containing parts that hybridize to an allele probe and an invader probe.

The test method of the present invention includes a step of determining the risk of antithyroid drug-induced agranulocytosis based on the above-mentioned test results of the polymorphism of the present invention. Therefore, in one embodiment, the step of determining the risk of antithyroid drug-induced agranulocytosis of the present invention includes a step of determining that the risk of antithyroid drug-induced agranulocytosis is high when the test subject has a risk allele of the polymorphism of the present invention, as compared to the test subject not having a risk allele.

According to the test method of the present invention, when, for example, a genotype for the polymorphism of the present invention in a test subject is a homozygote for risk allele, the frequency of developing or aggravating antithyroid drug-induced agranulocytosis is considered to be high as compared to a heterozygote for non-risk allele and risk allele, and a homozygote for non-risk allele.

Thus, in another embodiment, the step of determining the risk of antithyroid drug-induced agranulocytosis in the present invention includes a step of determining that the risk of antithyroid drug-induced agranulocytosis is high in the order of the test subject whose genotype for the polymorphism of the present invention is homozygote for risk allele, heterozygote for risk allele and non-risk allele, and homozygote for non-risk allele in the test subject.

In the test method of the present invention, when the number of tested polymorphism is high, the determination accuracy is also improved. Therefore, it is preferable to test not less than two polymorphisms selected from the polymorphisms of the present invention, and determine the risk of antithyroid drug-induced agranulocytosis. For example, two polymorphisms of the above-mentioned A) and B) are tested, and when both genotypes for the polymorphisms A) and B) are homozygotes for non-risk allele, the risk of antithyroid drug-induced agranulocytosis can be determined to be extremely low. Conversely, when genotypes for the polymorphisms A) and B) are homozygotes for risk allele, the risk of antithyroid drug-induced agranulocytosis can be determined to be extremely high.

More particularly, for example, as described in the following Example 4, when polymorphism in complete linkage of D'=1 with the polymorphism of the above-mentioned B) (rs2596487), that is, the polymorphism of the above-mentioned F), and the polymorphism of the above-mentioned E) (rs17576984) are tested, the risk of antithyroid drug-induced agranulocytosis can be determined to be high in the order of the rs2596487/rs17576984 score of the genotype of 2/2, 2/1, 1/2, 1/1, 2/0, 0/2, 1/0, 0/1, 0/0, wherein the homozygote for risk allele is score 2, heterozygote for risk allele and non-risk allele is score 1, and homozygote for non-risk allele is score 0.

In the test method of the present invention, the polymorphism of the above-mentioned A) or the polymorphism of B) is preferably tested, and two polymorphisms of the above-mentioned A) and B) are most preferably tested. In the step of testing the polymorphism of the present invention, not less than two polymorphisms of the above-mentioned F) alone may be tested. When not less than two polymorphisms are detected, they are desirably not completely linked, and most preferably not in a linkage disequilibrium state.

In one preferable embodiment of the test method of the present invention, polymorphism in linkage disequilibrium with the polymorphism of the above-mentioned A) at a linkage disequilibrium coefficient D' of not less than 0.8, and/or polymorphism in linkage disequilibrium with the polymorphism of the above-mentioned B) at a linkage disequilibrium coefficient D' of not less than 0.8 are/is tested. Examples of the polymorphism in linkage disequilibrium with the polymorphism of the above-mentioned A) at a linkage disequilibrium coefficient D' of not less than 0.8 include polymorphism completely linked with the polymorphism of the aforementioned A) and the like, and polymorphism at a position encoding the amino acid at position 74 (i.e., Leu) of HLA-DRB1*08:03 and HLA-DRB1*08:02 is particularly preferably tested. On the other hand, Examples of the polymorphism in linkage disequilibrium with the polymorphism of the above-mentioned B) at a linkage disequilibrium coefficient D' of not less than 0.8 include polymorphism completely linked with the polymorphism of the aforementioned B) and the like, and polymorphism at a position encoding the amino acid at position 116 (i.e., Phe) or the amino acid at position 158 (i.e., not Ala) of HLA-B*39:01 and HLA-B*38:02 is particularly preferably tested.

The present inventors have further found that HLA-B*39:01, HLA-DRB1*14:03, and HLA-DRB1*08:02 are strongly associated with antithyroid drug agranulocytosis. Furthermore, HLA-B*38:02 and HLA-DRB1*08:03 are also correlated with antithyroid drug-induced agranulocytosis. That is, when a test subject has one or more alleles selected from HLA-B*39:01, HLA-DRB1*14:03, HLA-DRB1*08:02, HLA-B*38:02 and HLA-DRB1*08:03, preferably alleles of HLA-B*39:01 and/or HLA-DRB1*14:03 and/or HLA-DRB1*08:02, the risk of antithyroid drug-induced agranulocytosis can be determined to be high. Therefore, the risk of antithyroid drug-induced agranulocytosis may be determined by, for example, testing, in combination with the test method of the present invention, whether the test subject has one or more alleles selected from HLA-B*39:01, HLA-DRB1*14:03, HLA-DRB1*08:02, HLA-B*38:02 and HLA-DRB1*08:03, preferably HLA-B*39:01 and/or HLA-DRB1*14:03 and/or HLA-DRB1*08:02.

A method of testing whether the test subject has HLA-B*39:01, HLA-B*38:02, HLA-DRB1*08:03, HLA-DRB1*14:03, or HLA-DRB1*08:02 is not particularly limited as long as it can distinguish HLA-B*39:01, HLA-B*38:02, HLA-DRB1*08:03, HLA-DRB1*14:03, or HLA-DRB1*08:02 allele from other HLA alleles and, for example, the above-mentioned polymorphism detection method can be used.

In the analysis of HLA allele, the corresponding gene sequence as a whole may be analyzed, or a part of the gene sequence may be analyzed. As a sample to be used for the analysis of HLA allele, those similar to the above-mentioned "sample derived from a test subject" can be preferably used.

HLA-DRB1*08:03, HLA-DRB1*14:03, and HLA-DRB1*08:02 are common in that they are polymorphisms accompanying amino acid substitution wherein the amino acid at position 74 of HLA-DRB1 protein is Leu. On the other hand, HLA-B*39:01 and HLA-B*38:02 are common in that they are polymorphisms accompanying amino acid substitution wherein the amino acid at position 116 of HLA-B protein is Phe. The present inventors have found that allele wherein the amino acid at position 74 of HLA-DRB1 protein is Leu (DRB1-Leu74), and an allele wherein the amino acid at position 116 of HLA-B protein is Phe (B-Phe116) are the risk alleles of antithyroid drug-induced agranulocytosis induced agranulocytosis. B-Phe116 is in linkage disequilibrium with polymorphism wherein the amino acid at position 158 of HLA-B protein is not Ala ($r^2$=0.92).

Therefore, the present invention also provides a test method for determining the risk of antithyroid drug-induced agranulocytosis, comprising

(1) a step of using a sample derived from a test subject and testing the following (a) and/or (b):

(a) whether the amino acid at position 74 of HLA-DRB1 protein is Leu
(b) whether the amino acid at position 116 of HLA-B protein is Phe, or the amino acid at position 158 of HLA-B protein is Ala, and

(2) a step of determining the risk of antithyroid drug-induced agranulocytosis based on the test results of (1) (hereinafter sometimes to be also referred to as the test method (2) of the present invention).

The test subject to be the target in the test method (2) of the present invention refers to hyperthyroidism patients, preferably Graves' disease patients, and is a human who is or is scheduled to be under medication of an antithyroid drug for the treatment or prophylaxis of the disease. The race of the test subject is not particularly limited and may be, for example, any of Mongoloid, Caucasoid and Negroid.

Examples of the (a) allele wherein the amino acid at position 74 of HLA-DR B1 protein is Leu to be the detection target include the aforementioned HLA-DRB1*08:03, HLA-DRB1*14:03, and HLA-DRB1*08:02 as well as, for example, HLA-DRB1*08:09 and the like. Examples of the (b) allele wherein the amino acid at position 116 of HLA-B protein is Phe (the amino acid at position 158 is not Ala) include HLA-B*39:01 and HLA-B*38:02 as well as, for example, HLA-B*37:01, HLA-B*39:04, HLA-B*67:01 and the like. Since the frequencies of these risk alleles is at a level that cannot be ignored not only in Mongoloid but also in Caucasoid and Negroid, the test method (2) of the present invention can be widely used irrespective of the race.

As "a sample derived from a test subject" to be the measurement target in the test method (2) of the present invention, a sample containing HLA-DRB1 and/or HLA-B protein(s) collected from the test subject may be used in addition to the biological sample containing genomic DNA, mRNA, total RNA of the test subject, which is described in the above-mentioned "test method of the present invention". That is, whether (a) the amino acid at position 74 of HLA-DRB1 protein is Leu, and/or whether (b) the amino acid at position 116 of HLA-B protein is Phe, or whether the amino acid at position 158 of HLA-B protein is Ala can be tested using an antibody or aptamer that specifically binds to a partial peptide containing Leu at position 74 of HLA-DRB1 protein, and/or an antibody or aptamer that specifically binds to a partial peptide containing Phe at position 116 of HLA-B protein or a partial peptide containing Ala at the 158-position, by detecting a complex of the HLA-DRB1 or HLA-B protein and the antibody or aptamer by an immunological method or a method analogous thereto.

As a result of the test, when (a) the amino acid at position 74 of HLA-DRB1 protein is Leu, and/or (b) the amino acid at position 116 of HLA-B protein is Phe, or the amino acid at position 158 is not Ala, the test subject can be determined to have a high risk of antithyroid drug-induced agranulocytosis.

In one embodiment, the present invention provides a kit for determination of the risk of antithyroid drug-induced agranulocytosis, comprising a polynucleotide capable of detecting a risk allele in
polymorphism present in the HLA region, which is at least one selected from the group consisting of

A) polymorphism at the 501st nucleotide in the nucleotide sequence shown in SEQ ID NO: 1 (G>T*),
B) polymorphism at the 201st nucleotide in the nucleotide sequence shown in SEQ ID NO: 2 (C>T*),
C) polymorphism at the 501st nucleotide in the nucleotide sequence shown in SEQ ID NO: 3 (C>T*),
D) polymorphism at the 501st nucleotide in the nucleotide sequence shown in SEQ ID NO: 4 (T*>G),
E) polymorphism at the 501st nucleotide in the nucleotide sequence shown in SEQ ID NO: 5 (C>T*),
wherein parentheses show reference allele>variant allele, * is risk allele, G, A, T and C are guanine, adenine, thymine and cytosine, respectively, and
F) polymorphism in linkage disequilibrium with the polymorphism of any of the above-mentioned A) - E), the linkage disequilibrium showing a linkage disequilibrium coefficient D' of not less than 0.8
(that is, the polymorphism of the present invention)
(hereinafter to be also indicated as the evaluation kit of the present invention).

A polynucleotide capable of detecting a risk allele of the polymorphism of the present invention is useful for determining the risk of antithyroid drug-induced agranulocytosis of the test subject. Therefore, the evaluation kit of the present invention contains a polynucleotide capable of detecting a risk allele of the polymorphism of the present invention.

Such polynucleotide is capable of detecting, for example, in the polymorphism of the above-mentioned A), the presence of a sequence which is the nucleotide sequence shown in SEQ ID NO: 1 wherein the 501st nucleotide is T (A in complementary chain sequence) and, in the polymorphism of the above-mentioned B), the presence of a sequence which is the nucleotide sequence shown in SEQ ID NO: 2 wherein the 201st nucleotide is T (A in complementary chain sequence).

The evaluation kit of the present invention preferably contains a polynucleotide capable of detecting a risk allele of the polymorphism of the above-mentioned A) and/or B).

In preferable one embodiment of the evaluation kit of the present invention, the kit contains a polynucleotide capable of detecting a risk allele in polymorphism in linkage disequilibrium with the polymorphism of the above-mentioned A) at a linkage disequilibrium coefficient D' of not less than 0.8, and/or polymorphism in linkage disequilibrium with the polymorphism of the above-mentioned B) at a linkage disequilibrium coefficient D' of not less than 0.8. Examples of the polymorphism in linkage disequilibrium with the polymorphism of the above-mentioned A) at a linkage disequilibrium coefficient D' of not less than 0.8 include polymorphism in complete linkage with the polymorphism of the aforementioned A) and the like, and a polynucleotide capable of detecting polymorphism at a position encoding the amino acid at position 74 of HLA-DRB1*08:03 and HLA-DRB1*08:02 (i.e., Leu) is particularly preferably contained. Examples of the polymorphism in linkage disequilibrium with the polymorphism of the above-mentioned B) at a linkage disequilibrium coefficient D' of not less than 0.8 include polymorphism in complete linkage with the polymorphism of the aforementioned B) and the like, and a polynucleotide capable of detecting polymorphism at a position encoding the amino acid at position 116 of HLA-B*39:01 and HLA-B*38:02 (i.e., Phe) or the amino acid at position 158 of HLA-B*39:01 and HLA-B*38:02 (i.e., not Ala) is particularly preferably contained.

The evaluation kit of the present invention desirably contains a polynucleotide capable of detecting a non-risk allele of the polymorphism of the present invention. Such polynucleotide is capable of detecting, for example, in the polymorphism of the above-mentioned A), the presence of a sequence which is the nucleotide sequence shown in SEQ ID NO: 1 wherein the 501st nucleotide is G (C in complementary chain sequence) and, in the polymorphism of the above-mentioned B), the presence of a sequence which is the nucleotide sequence shown in SEQ ID NO: 2 wherein the 201st nucleotide is C (G in complementary chain sequence). In the following, in the polymorphism of the present invention, a polynucleotide capable of detecting a risk allele and a polynucleotide capable of detecting a non-risk allele are also collectively indicated as "the polynucleotide capable of detecting the polymorphism of the present invention".

To be specific, the polynucleotide capable of detecting the polymorphism of the present invention is used as a primer or probe in known polymorphism analysis method such as the above-mentioned polymorphism detection methods, for example, RFLP method, PCR-SSCP method, ASO hybridization, direct sequencing method, ARMS method, denaturing gradient gel electrophoresis, RNase A cleavage method, chemical cleavage method, DOL method, TaqMan PCR method, invader method, MALDI-TOF/MS METHOD, TDI method, molecular beacon method, dynamic allele-specific hybridization method, padlock probe method, UCAN method, nucleic acid hybridization method using DNA chip or DNA microarray, ECA method and the like.

When the polynucleotide capable of detecting the polymorphism of the present invention is a primer, the primer may be any as long as it is designed to be able to specifically amplify the region of genomic DNA (or mRNA) containing the nucleotide of the polymorphic site of the present invention. When the polynucleotide capable of detecting the polymorphism of the present invention is a probe, the probe may be any as long as it is designed to be able to hybridize to a region of a genomic DNA (or mRNA) containing a nucleotide of the polymorphic site of the present invention under stringent conditions.

In the present specification, the "stringent conditions" refers to conditions under which polynucleotides having a nucleotide sequence homology of not less than about 90%, preferably not less than about 95%, particularly preferably not less than about 96, 97, 98, 99%, most preferably 100%, can hybridize to each other. Stringency can be controlled by appropriately changing the salt concentration, temperature and the like during hybridization reaction and washing, and those of ordinary skill in the art can easily set preferable conditions.

The length of the polynucleotide capable of detecting the polymorphism of the present invention is not particularly limited as long as it can detect a DNA fragment in the HLA region comprising 10 - 200 continuous nucleotide sequences containing the polymorphic site. Those of ordinary skill in the art can appropriately select the length according to the use of the polynucleotide.

When the polynucleotide of the present invention is used as a primer, it has a nucleotide length of, for example, 10 - 200 bp, preferably 15 - 100 bp, more preferably 15 - 35 bp. The length of DNA that the primer can amplify is, for example, 15 - 1000 bp, preferably 20 - 500 bp, more preferably 20 - 200 bp.

When the polynucleotide of the present invention is used as a probe, it has a nucleotide length of, for example, 10 - 200 bp, preferably 15 - 100 bp, more preferably 15 - 35 bp.

When the polynucleotide capable of detecting the polymorphism of the present invention is used as a primer, the primer may contain an additional sequence (sequence not complementary to genomic DNA (or mRNA)), for example, a linker sequence, suitable for detecting the polymorphism.

The primer may be labeled with a suitable label, for example, radioisotope (e.g., $^{125}I$, $^{131}I$, $^{3}H$, $^{14}C$ and the like), enzyme (e.g., β-galactosidase, β-glucosidase, alkaline phosphatase, peroxidase, malic acid dehydrogenase and the like), fluorescent substance (e.g., fluorescamine, fluorescein isothiocyanate, Cy3, Cy5 and the like), luminescence substance (e.g., luminol, luminol derivative, luciferin, lucigenin and the like) and the like.

When the polynucleotide capable of detecting the polymorphism of the present invention is used as a probe, the probe may contain an additional sequence (sequence not complementary to genomic DNA (or mRNA)) suitable for detecting the polymorphism. For example, a probe used for the Invader probe method may contain an additional sequence called flap at the 5'-terminal of the nucleotide of polymorphic site.

The probe may be labeled with a suitable label, for example, radioisotope (e.g., $^{125}I$, $^{131}I$, $^{3}H$, $^{14}C$ and the like), enzyme (e.g., β-galactosidase, β-glucosidase, alkaline phosphatase, peroxidase, malic acid dehydrogenase and the like), fluorescent substance (e.g., fluorescamine, fluorescein isothiocyanate, Cy3, Cy5 and the like), luminescence substance (e.g., luminol, luminol derivative, luciferin, lucigenin and the like) and the like. Alternatively, a quencher (quenching substance) that absorbs fluorescence energy emitted by a fluorescent substance may be further bonded in the vicinity of the fluorescent substance (e.g., FAM, VIC etc.). In such embodiment, the fluorescent substance and the quencher are separated during detection reaction and fluorescence is detected.

The above-mentioned probe and/or primer are/is dissolved separately (or in a mixed state when possible) in water or a suitable buffer (e.g., TE buffer etc.) to a suitable concentration (e.g., 2 - 20× concentration of 1 - 50 μM and the like), and preserved at about -20°C.

The evaluation kit of the present invention contains at least one kind of the polynucleotide capable of detecting the polymorphism of the present invention (capable of detecting at least risk allele). Since a larger number of polymorphism to be detected improves the determination accuracy, two or more kinds of the polynucleotide capable of detecting the polymorphism of the present invention (capable of detecting at least risk allele) are preferably contained.

The polynucleotide capable of detecting the polymorphism of the present invention may be DNA or RNA, and may be single strand or double strand. In the case of a double strand, the nucleic acid may be a double-stranded DNA, a double-stranded RNA, or a DNA/RNA hybrid. Therefore, when a nucleic acid having a certain nucleotide sequence is described herein, it should be understood that, unless otherwise indicated, the term nucleic acid is used with a meaning that encompasses all of single-stranded nucleic acids having the nucleotide sequence, single-stranded nucleic acids having a complementary sequence to the nucleotide sequence, and double-stranded nucleic acids which are hybrids thereof.

The above-mentioned polynucleotide can be synthesized, for example, based on the information of nucleotide sequence shown by SEQ ID NO: 1 - 5 according to a conventional method using a DNA/RNA automatic synthesizer.

The "test subject" to be the target for the evaluation kit of the present invention refers to a human who is a hyperthyroidism patient, preferably Graves' disease patient, and is or is scheduled to be under medication of an antithyroid drug for the treatment or prophylaxis of the disease. While the race of the test subject is not particularly limited, preferred are eastern Asians, more preferred are Japanese people.

The evaluation kit of the present invention may further contain, according to the polymorphism detection method, other constituent elements necessary for performing the method. For example, when the kit is for polymorphism detection by the TaqMan PCR method, the kit may further contain, though not limited to, 10xPCR reaction buffer, 10x$MgCl_2$ aqueous solution, 10xdNTPs aqueous solution, Taq DNA polymerase (5 U/μL), diagnostic criteria table for the risk of antithyroid drug-induced agranulocytosis, manual explaining the kit operation method and the like.

The present invention also provides a kit for determination of the risk of antithyroid drug-induced agranulocytosis, comprising a substance capable of identifying the following (a) and/or (b):

(a) the amino acid at position 74 of HLA-DRB1 protein is Leu
(b) the amino acid at position 116 of HLA-B protein is Phe, or the amino acid at position 158 is Ala of HLA-B protein (the evaluation kit (2) of the present invention).

Examples of the substance capable of identifying that the amino acid at position 74 of HLA-DRB1 protein is Leu, the amino acid at position 116 of HLA-B protein is Phe, or the amino acid at position 158 is Ala include a polynucleotide capable of detecting a partial nucleotide sequence containing a codon encoding the amino acid at position 74 of HLA-DRB1 protein, which is in HLA-DRB1 gene or mRNA, a polynucleotide capable of detecting a partial nucleotide sequence containing a codon encoding the amino acid at position 116 of HLA-B protein, which is in HLA-B gene or mRNA, and the like. These polynucleotides can be designed and prepared by a method similar to that described in detail in the above-mentioned "evaluation kit of the present invention".

Examples of other substance capable of identifying that the amino acid at position 74 of HLA-DRB1 protein is Leu, the amino acid at position 116 of HLA-B protein is Phe, or the amino acid at position 158 is Ala include an antibody or aptamer that specifically binds to a partial peptide containing Leu at position 74 of HLA-DRB1 protein, an antibody or aptamer that specifically binds to a partial peptide containing Phe at position 116 or a partial peptide containing Ala at position 158 of HLA-B protein and the like. Such antibody or aptamer can be obtained by a well-known method.

The "test subject" to be the target for the evaluation kit (2) of the present invention refers to a human who is a hyperthyroidism patient, preferably Graves' disease patient, and is or is scheduled to be under medication of an antithyroid drug for the treatment or prophylaxis of the disease. The race of the test subject is not particularly limited and, for example, it may be any of Mongoloid, Caucasoid and Negroid.

The evaluation kit (2) of the present invention may further contain, according to the polymorphism detection method, other constituent elements necessary for performing the method. For example, when the kit is for polymorphism detection by the TaqMan PCR method, the evaluation kit of the present invention, it can contain constituent elements similar to those of the above-mentioned "evaluation kit of the present invention". On the other hand, when the kit is for polymorphism detection by an antibody or aptamer, the kit can contain reagents, containers and the like generally used for immunological measurement methods, such as reaction buffer, secondary antibody, labeling substance, microplate and the like, as further constituent elements.

The present invention is explained in more detail in the following by referring to Examples, which are not to be construed as limitative.

Examples

[Example 1]

Search for SNP correlated to antithyroid drug-induced agranulocytosis

(1) test subject

DNA samples of patients diagnosed with antithyroid drug-induced agranulocytosis (not more than 500/μL) were collected for 63 cases from Kuma Hospital (Kobe, Japan) and 52 cases from Ito Hospital (Tokyo, Japan) (115 cases in total). Of the 115 cases, 113 cases were diagnosed with Graves' disease, and the remaining 2 cases were diagnosed with painless thyroiditis and hyperthyroidism, and they were under treatment with an antithyroid drug. The antithyroid drugs used for the treatment were methimazole (95 cases) and propylthiouracil (hereinafter to be also indicated as PTU, 20 cases). The patients' clinical information such as age, sex, granulocyte number and the like were collected by thyroid gland medical specialists in each institution.

As a control group, DNA samples of 1,937 cases collected for genome cohort study (hereinafter to be also indicated as Nagahama cohort) in Nagahama, Shiga, were used. The characteristics of patients used for this test are shown in the following Table 1.

Table 1

| | cases | controls |
|---|---|---|
| first set | | |
| institution | Kuma Hospital | Kyoto University |
| sample number | 63 | 1562 |
| age (mean±S.D.) | 41.2±13.9 | 53.5±13.4 |
| female ratio | 0.89 | 0.65 |
| genotyping array | Illumina Infinium HumanOmni2.5-8 | Illumina Infinium HumanOmni2.5-4 |
| disease name | Graves' disease: 62 samples<br>painless thyroiditis: 1 sample | |
| antithyroid drug | methimazole: 51 samples PTU: 12 samples | - |
| granulocyte number (mean) on diagnosis | 0-492 (120) | - |
| second set | | |
| institution | Ito Hospital | Kyoto University |
| sample number | 52 | 375 |
| age (mean±S.D.) | 40.4±12.3 | 52.9±12.3 |
| female ratio | 1 | 0.69 |

(continued)

| | | |
|---|---|---|
| second set | | |
| genotyping array | Illumina Infinium HumanOmni2.5-8 | Illumina Infinium HumanOmni2.5-8 |
| disease name | Graves' disease: 51 samples hyperthyroidism: 1 sample | |
| antithyroid drug | methimazole: 44 samples PTU: 8 samples | - |
| granulocyte number (mean) on diagnosis | 0-448 (35) | - |

DNA samples of 89 Graves' disease patients were obtained from Kyoto University. This study was approved by the Ethics Committee of each Research Institute, and informed consent was obtained from all test subjects.

(2) genome wide association study (GWAS)

GWAS was performed in 2 sets. In the first set, DNA samples of 63 cases obtained from Kuma Hospital were used as a case group, and genotyped using Illumina infinium HumanOmni 2.5-8 v1.0 DNA analysis kit (Illumina, Inc). As a control group, DNA samples of 1,562 cases obtained from Nagahama cohort were genotyped using Illumina Infinium HumanOmni 2.5-4 v1.0 DNA analysis kit (Illumina, Inc).

In the second set, DNA samples of 52 cases obtained from Ito Hospital were used as a case group, DNA samples of 375 cases obtained from Nagahama cohort were used as a control group, and the both were genotyped using Illumina infinium HumanOmni 2.5-8 v1.0 DNA analysis kit (Illumina, Inc).

After genotyping using SNP array, DNA samples (12 cases) with call success rate of less than 95%, DNA samples (122 cases) in high consanguinity (PI_HAT$\geq$0.35 by PLINK software) with other samples, and DNA samples (5 cases) deviated from the Asian cluster by main component analysis were removed. As a result, after quality control, the DNA sample in the first set consisted of case group: 63 cases and control group: 1,445 cases, and the DNA samples in the second set consisted of case group: 52 cases and control group: 353 cases.

As the SNP marker, 2,635,435 SNPs common to two arrays were noted and SNP markers showing a call success rate of not less than 95%, minor allele frequency of not less than 0.01 in case group or control group, and Hardy-Weinberg equilibrium test p value>$1.0\times10^{-7}$ were selected from them. As a result, 1,223,017 SNPs (first set) and 1,246,969 SNPs (second set) in total were used for the analysis.

The DNA samples of Graves' disease patients were used for genotyping of SNP in association with agranulocytosis by capillary sequencing using 3730x1 DNA analyzer (Life Technologies).

(3) statistical analysis

Statistical analysis of 2 sets of GWAS and integrated evaluation was performed by chi-squared test or Fisher's exact probability test (Fisher's exact test) using PLINK software or R software. A heterogeneity test between the tests was performed using a Breslow-Day test. Fisher's exact probability test was used when expectation in the 2x2 contingency table contained not more than 5. Multiple logistic regression analysis was performed to examine independency of SNP in the HLA region. The GWAS significant level after Bonferroni's correction in the multiple test was set to $5.0\times10^{-8}$. The interaction between the associated SNPs was evaluated by a conventionally-known method (Andersson et al., European journal of epidemiology 2005; 20: 575-9). Haploview version 4.1 software was used for the analysis of LD values between SNPs and drawing of a linkage disequilibrium map.

(4) results

The first set (case group: 63 cases and control group: 1,445 cases) and the second set (case group: 52 cases and control group: 353 cases) were subjected to a case-control study to identify 191 SNPs, showing extremely strong association with antithyroid drug-induced agranulocytosis, in the human HLA region on the short arm of human chromosome 6 6p21.3 (Fig. 1). Structuring of the population was not observed. The identified 191 SNPs are shown in the following Tables 2 - 6 together with p value, odds ratio (OR), 95% confidence interval (CI) obtained by integrated analysis of the two sets.

Table 2

| rs number | chromosome | position | allele (A1/A2) | gene | integrated analysis | | | |
|---|---|---|---|---|---|---|---|---|
| | | | | | A2 frequency | | p value | OR (95%CI) |
| | | | | | patients | controls | | |
| rs1633041 | 6 | 29841202 | G/A | 3.8-1.5 | 0.322 | 0.152 | 1.14E-11 | 2.66(1.98-3.55) |
| rs1737041 | 6 | 29844208 | C/A | HCP5P14 | 0.322 | 0.154 | 2.82E-11 | 2.6(1.95-3.48) |
| rs1002046 | 6 | 29861995 | C/T | HCG4P10 | 0.322 | 0.154 | 3.01E-11 | 2.6(1.94-3.48) |
| snp1 | 6 | 29867289 | G/A | HCG4 | 0.322 | 0.154 | 2.82E-11 | 2.6(1.95-3.48) |
| rs1610644 | 6 | 29867424 | C/T | HCG4 | 0.322 | 0.154 | 3.01E-11 | 2.6(1.94-3.48) |
| snp2 | 6 | 29867864 | G/A | HCG4 | 0.322 | 0.152 | 1.56E-11 | 2.64(1.97-3.53) |
| rs1633011 | 6 | 29870672 | C/T | HCP5P13 | 0.314 | 0.153 | 2.11E-10 | 2.53(1.88-3.4) |
| rs1630969 | 6 | 29872980 | G/A | HCP5P13 | 0.286 | 0.147 | 2.51E-08 | 2.32(1.71-3.15) |
| snp3 | 6 | 29877476 | A/C | RPL7AP7 | 0.322 | 0.154 | 2.34E-11 | 2.62(1.95-3.5) |
| rs1632988 | 6 | 29880374 | G/A | RPL7AP7 | 0.289 | 0.145 | 5.42E-09 | 2.39 (1.77-3.23) |
| rs1632987 | 6 | 29880528 | C/G | RPL7AP7 | 0.322 | 0.151 | 1.09E-11 | 2.66(1.99-3.56) |
| rs1736971 | 6 | 29884301 | G/T | MICG | 0.322 | 0.152 | 1.48E-11 | 2.64(1.97-3.53) |
| rs1736969 | 6 | 29884369 | A/T | MICG | 0.325 | 0.154 | 1.65E-11 | 2.64(1.97-3.53) |
| rs1610663 | 6 | 29886605 | C/T | MICG | 0.326 | 0.166 | 5.51E-10 | 2.44(1.82-3.26) |
| rs1610699 | 6 | 29886966 | T/G | MICG | 0.326 | 0.165 | 5.03E-10 | 2.44(1.83-3.26) |
| rs11753629 | 6 | 29890310 | G/A | MICG | 0.346 | 0.535 | 3.49E-08 | 0.46(0.35-0.61) |
| rs1736959 | 6 | 29890449 | C/T | MICG | 0.316 | 0.142 | 1.72E-12 | 2.79(2.07-3.74) |
| rs1736957 | 6 | 29890612 | T/C | MICG | 0.322 | 0.154 | 2.62E-11 | 2.61(1.95-3.49) |
| rs1620173 | 6 | 29893128 | A/C | MICG | 0.322 | 0.155 | 4.48E-11 | 2.58(1.93-3.45) |
| rs1619379 | 6 | 29893214 | C/T | MICG | 0.365 | 0.194 | 4.75E-10 | 2.39(1.8-3.17) |
| rs2735028 | 6 | 29893517 | C/T | MICG | 0.326 | 0.165 | 5.17E-10 | 2.44(1.83-3.26) |
| rs1049033 | 6 | 29905618 | C/T | HLA-G | 0.302 | 0.138 | 2.77E-11 | 2.7(1.99-3.65) |
| rs11753296 | 6 | 29909089 | G/A | HCGVIII-2 | 0.305 | 0.52 | 4.17E-10 | 0.41(0.3-0.54) |
| rs9404952 | 6 | 29912144 | G/A | HCGVIII-2 | 0.635 | 0.802 | 1.32E-09 | 0.43(0.32-0.57) |
| rs2394178 | 6 | 29912824 | G/A | HCGVIII-2 | 0.302 | 0.517 | 5.24E-10 | 0.4(0.3-0.54) |
| rs2394180 | 6 | 29913178 | T/C | HCGVIII-2 | 0.313 | 0.518 | 1.78E-09 | 0.42(0.32-0.56) |
| rs2735014 | 6 | 29913788 | C/A | HCGVIII-2 | 0.307 | 0.515 | 1.29E-09 | 0.42(0.31-0.56) |
| rs2735009 | 6 | 29915832 | G/A | HCGVIII-2 | 0.307 | 0.511 | 2.46E-09 | 0.42(0.32-0.57) |
| rs2743937 | 6 | 29915902 | T/C | HCGVIII-2 | 0.313 | 0.52 | 1.15E-09 | 0.42(0.32-0.56) |
| rs2735005 | 6 | 29916443 | G/A | HCGVIII-2 | 0.351 | 0.552 | 3.46E-09 | 0.44(0.33-0.58) |

(continued)

| rs number | chromosome | position | allele (A1/A2) | gene | integrated analysis | | | |
|---|---|---|---|---|---|---|---|---|
| | | | | | A2 frequency | | p value | OR (95%CI) |
| | | | | | patients | controls | | |
| rs2735003 | 6 | 29916613 | T/G | HCGVIII-2 | 0.307 | 0.52 | 4.65E-10 | 0.41(0.31-0.55) |
| rs9258537 | 6 | 29916840 | G/A | HCGVIII-2 | 0.307 | 0.52 | 4.40E-10 | 0.41(0.31-0.55) |
| rs2523777 | 6 | 29917066 | C/T | HCGVIII-2 | 0.307 | 0.52 | 4.42E-10 | 0.41(0.31-0.55) |
| rs1611133 | 6 | 29917361 | C/T | HCGVIII-2 | 0.322 | 0.153 | 2.32E-11 | 2.62(1.96-3.5) |
| rs2523774 | 6 | 29917557 | C/T | HCGVIII-2 | 0.329 | 0.532 | 2.55E-09 | 0.43(0.32-0.57) |
| rs2523769 | 6 | 29919001 | A/C | HCGVIII-2 | 0.357 | 0.557 | 3.16E-09 | 0.44(0.33-0.58) |
| rs1611750 | 6 | 29922757 | T/G | MICF | 0.322 | 0.155 | 4.70E-11 | 2.58(1.93-3.45) |
| rs9261257 | 6 | 30130404 | G/A | ZNRD1 | 0.183 | 0.078 | 3.00E-08 | 2.65(1.85-3.78) |
| rs34632463 | 6 | 31102738 | A/C | C6orf205 | 0.241 | 0.108 | 1.15E-09 | 2.62(1.9-3.62) |
| rs1265100 | 6 | 31213289 | A/G | PSORS1C1 | 0.474 | 0.289 | 3.26E-09 | 2.21(1.69-2.9) |
| snp4 | 6 | 31407142 | T/C | HLA-B | 0.22 | 0.071 | 5.14E-15 | 3.69(2.61-5.23) |
| snp5 | 6 | 31427851 | C/A | HLA-B | 0.208 | 0.053 | 1.22E-20 | 4.66(3.28-6.63) |
| snp6 | 6 | 31428376 | C/T | HLA-B | 0.219 | 0.055 | 1.81E-22 | 4.81(3.41-6.79) |
| rs41560220 | 6 | 31431854 | C/T | HLA-B | 0.224 | 0.05 | 2.10E-26 | 5.44(3.85-7.68) |
| rs2596487 | 6 | 31433035 | C/T | HLA-B | 0.219 | 0.049 | 2.39E-25 | 5.39(3.79-7.65) |

Table 3

| rs number | chromosome | position | allele (A1/A2) | gene | integrated analysis | | | |
|---|---|---|---|---|---|---|---|---|
| | | | | | A2 frequency | | p value | OR (95%CI) |
| | | | | | patients | controls | | |
| snp7 | 6 | 31463601 | A/G | LOC442200 | 0.128 | 0.04 | 5.54E-10 | 3.54(2.31-5.41) |
| rs3828875 | 6 | 31487147 | C/T | MICA | 0.145 | 0.043 | 5.00E-12 | 3.77(2.52-5.64) |
| rs2273612 | 6 | 31733313 | C/T | APOM | 0.052 | 0.008 | 3.00E-10 | 6.77(3.41-13.45) |
| rs11964779 | 6 | 31819603 | A/C | MSH5 | 0.052 | 0.008 | 6.60E-10 | 6.53(3.3-12.93) |
| rs11967206 | 6 | 31840658 | C/T | MSH5 | 0.052 | 0.008 | 6.22E-10 | 6.54(3.3-12.96) |
| rs707934 | 6 | 31843610 | C/T | C6orf27 | 0.061 | 0.009 | 5.50E-12 | 6.99(3.68-13.25) |
| rs17207552 | 6 | 31873035 | G/C | LSM2 | 0.053 | 0.008 | 5.13E-10 | 6.59(3.33-13.06) |
| rs34467149 | 6 | 31901695 | C/T | HSPA1B | 0.052 | 0.009 | 1.24E-09 | 6.33(3.21-12.5) |
| rs3998219 | 6 | 31970367 | C/T | EHMT2 | 0.109 | 0.03 | 1.69E-10 | 4(2.53-6.33) |
| rs10947230 | 6 | 32132373 | C/T | TNXB | 0.143 | 0.049 | 1.05E-09 | 3.25(2.18-4.85) |
| rs7774197 | 6 | 32154253 | A/C | TNXB | 0.143 | 0.054 | 2.76E-08 | 2.93(1.97-4.36) |
| rs204992 | 6 | 32264886 | G/A | PBX2 | 0.219 | 0.078 | 3.37E-13 | 3.32(2.36-4.66) |
| rs204991 | 6 | 32269344 | T/C | GPSM3 | 0.387 | 0.221 | 6.50E-09 | 2.23(1.69-2.94) |
| rs204990 | 6 | 32269408 | C/A | GPSM3 | 0.387 | 0.223 | 1.31E-08 | 2.19(1.66-2.89) |
| rs17604492 | 6 | 32286548 | C/T | NOTCH4 | 0.226 | 0.078 | 1.17E-14 | 3.45(2.48-4.81) |
| rs8192569 | 6 | 32298462 | G/A | NOTCH4 | 0.369 | 0.179 | 2.38E-12 | 2.69(2.02-3.58) |
| rs375244 | 6 | 32299435 | G/A | NOTCH4 | 0.422 | 0.668 | 3.14E-14 | 0.36(0.28-0.48) |
| rs3134930 | 6 | 32299598 | C/T | NOTCH4 | 0.417 | 0.193 | 5.06E-16 | 2.99(2.27-3.94) |
| rs8192564 | 6 | 32299800 | G/A | NOTCH4 | 0.239 | 0.086 | 1.95E-14 | 3.33(2.41-4.61) |
| snp8 | 6 | 32301197 | C/T | NOTCH4 | 0.243 | 0.093 | 2.14E-13 | 3.15(2.29-4.35) |
| snp9 | 6 | 32302999 | G/A | NOTCH4 | 0.239 | 0.087 | 2.89E-14 | 3.31(2.39-4.57) |
| rs365053 | 6 | 32303966 | T/G | NOTCH4 | 0.583 | 0.368 | 8.12E-11 | 2.4(1.83-3.15) |
| snp10 | 6 | 32304044 | C/T | NOTCH4 | 0.23 | 0.061 | 3.02E-22 | 4.62(3.3-6.46) |
| snp11 | 6 | 32304227 | G/T | NOTCH4 | 0.235 | 0.073 | 4.09E-18 | 3.92(2.82-5.45) |
| rs17576984 | 6 | 32320963 | C/T | LOC401252 | 0.456 | 0.167 | 1.15E-27 | 4.17(3.17-5.49) |
| rs6936204 | 6 | 32325070 | T/C | LOC401252 | 0.709 | 0.912 | 4.01E-23 | 0.24(0.17-0.32) |
| rs3115572 | 6 | 32328462 | C/G | LOC401252 | 0.587 | 0.788 | 1. 36E-12 | 0.38(0.29-0.5) |
| rs3130316 | 6 | 32329206 | T/C | LOC401252 | 0.592 | 0.787 | 9.15E-12 | 0.39(0.3-0.52) |
| rs3096686 | 6 | 32338075 | C/G | LOC401252 | 0.407 | 0.224 | 3.63E-10 | 2.37 (1.8-3.13) |
| rs9268055 | 6 | 32338586 | T/C | LOC401252 | 0.412 | 0.22 | 2.16E-11 | 2.49(1.89-3.28) |

(continued)

| rs number | chromosome | position | allele (A1/A2) | gene | integrated analysis | | | |
|---|---|---|---|---|---|---|---|---|
| | | | | | A2 frequency | | p value | OR (95%CI) |
| | | | | | patients | controls | | |
| rs3096681 | 6 | 32343155 | A/G | LOC401252 | 0.417 | 0.219 | 4.85E-12 | 2.55(1.94-3.35) |
| rs3132931 | 6 | 32343873 | T/G | LOC401252 | 0.413 | 0.211 | 9.11E-13 | 2.64(2-3.47) |
| rs3115560 | 6 | 32344120 | G/A | LOC401252 | 0.417 | 0.219 | 4.77E-12 | 2.55(1.94-3.36) |
| rs3096673 | 6 | 32345991 | T/C | LOC401252 | 0.417 | 0.219 | 4.44E-12 | 2.56(1.94-3.36) |
| rs3096674 | 6 | 32346197 | G/A | LOC401252 | 0.413 | 0.212 | 1.57E-12 | 2.61(1.98-3.43) |
| rs3115557 | 6 | 32347629 | C/T | LOC401252 | 0.413 | 0.213 | 1.71E-12 | 2.6(1.98-3.43) |
| rs3130340 | 6 | 32352605 | T/C | LOC401252 | 0.417 | 0.218 | 3.04E-12 | 2.58(1.96-3.39) |
| rs3115553 | 6 | 32353805 | C/T | LOC401252 | 0.417 | 0.218 | 2.98E-12 | 2.58(1.96-3.39) |
| snp12 | 6 | 32353814 | C/T | LOC401252 | 0.291 | 0.086 | 4.84E-24 | 4.35(3.2-5.92) |
| rs3115552 | 6 | 32354134 | C/T | LOC401252 | 0.417 | 0.219 | 4.23E-12 | 2.56(1.95-3.36) |
| rs9268125 | 6 | 32360656 | T/C | C6orf10 | 0.412 | 0.214 | 3.97E-12 | 2.58(1.96-3.39) |
| rs9268131 | 6 | 32362430 | A/G | C6orf10 | 0.413 | 0.213 | 1.71E-12 | 2.6(1.98-3.43) |
| rs9268135 | 6 | 32363208 | A/G | C6orf10 | 0.411 | 0.209 | 1.58E-12 | 2.64(2-3.49) |
| rs9268137 | 6 | 32363247 | G/A | C6orf10 | 0.413 | 0.211 | 9.38E-13 | 2.64(2-3.47) |
| rs7751896 | 6 | 32363388 | G/A | C6orf10 | 0.417 | 0.219 | 4.66E-12 | 2.55(1.94-3.36) |

[Table 4]

| rs number | chromosome | position | allele (A1/A2) | gene | integrated analysis | | | |
|---|---|---|---|---|---|---|---|---|
| | | | | | A2 frequency | | p value | OR (95%CI) |
| | | | | | patients | controls | | |
| rs9268142 | 6 | 32364396 | T/C | C6orf10 | 0.413 | 0.212 | 1.49E-12 | 2.61(1.98-3.44) |
| rs6935269 | 6 | 32368328 | T/C | C6orf10 | 0.417 | 0.217 | 2.92E-12 | 2.58(1.96-3.39) |
| rs3749966 | 6 | 32369485 | T/C | C6orf10 | 0.417 | 0.217 | 2.92E-12 | 2.58(1.96-3.39) |
| rs11751697 | 6 | 32374403 | C/T | C6orf10 | 0.413 | 0.206 | 2.07E-13 | 2.71(2.06-3.56) |
| rs7750783 | 6 | 32376058 | C/T | C6orf10 | 0.413 | 0.212 | 1.41E-12 | 2.61(1.99-3.44) |
| rs6909427 | 6 | 32376679 | T/G | C6orf10 | 0.417 | 0.217 | 2.46E-12 | 2.59(1.97-3.4) |
| rs3864300 | 6 | 32379785 | G/T | C6orf10 | 0.417 | 0.219 | 4.62E-12 | 2.55(1.94-3.36) |
| rs9268168 | 6 | 32380488 | C/T | C6orf10 | 0.417 | 0.217 | 2.86E-12 | 2.58(1.96-3.39) |
| rs6457536 | 6 | 32381743 | A/G | C6orf10 | 0.417 | 0.217 | 2.77E-12 | 2.58(1.96-3.39) |
| rs7341328 | 6 | 32383172 | G/A | C6orf10 | 0.417 | 0.217 | 2.64E-12 | 2.58(1.96-3.4) |
| rs9268192 | 6 | 32385189 | C/T | C6orf10 | 0.417 | 0.219 | 4.66E-12 | 2.55(1.94-3.36) |
| rs9268200 | 6 | 32386648 | C/T | C6orf10 | 0.417 | 0.219 | 4.85E-12 | 2.55(1.94-3.35) |
| rs9268202 | 6 | 32387318 | C/T | C6orf10 | 0.403 | 0.213 | 2.90E-11 | 2.5(1.89-3.3) |
| rs9501173 | 6 | 32387880 | G/A | C6orf10 | 0.413 | 0.208 | 3.92E-13 | 2.67(2.03-3.52) |
| rs1018433 | 6 | 32389488 | A/T | C6orf10 | 0.417 | 0.219 | 4.51E-12 | 2.55(1.94-3.36) |
| rs6909790 | 6 | 32390957 | A/G | C6orf10 | 0.417 | 0.219 | 4.62E-12 | 2.55(1.94-3.36) |
| rs6915455 | 6 | 32391472 | G/A | C6orf10 | 0.417 | 0.219 | 4.33E-12 | 2.56(1.94-3.36) |
| rs3749967 | 6 | 32391822 | T/C | C6orf10 | 0.413 | 0.206 | 2.20E-13 | 2.7(2.05-3.56) |
| rs9469099 | 6 | 32416886 | G/A | C6orf10 | 0.292 | 0.086 | 3.80E-24 | 4.41(3.24-6.01) |
| snp13 | 6 | 32423700 | G/A | C6orf10 | 0.274 | 0.083 | 1.27E-21 | 4.14(3.03-5.66) |
| rs2395148 | 6 | 32429532 | G/T | C6orf10 | 0.291 | 0.084 | 9.94E-25 | 4.46(3.27-6.06) |
| rs3129942 | 6 | 32446261 | G/T | C6orf10 | 0.269 | 0.127 | 5.19E-09 | 2.54(1.84-3.5) |
| snp14 | 6 | 32452309 | G/A | C6orf10 | 0.113 | 0.035 | 5.87E-09 | 3.47(2.23-5.42) |
| rs9268460 | 6 | 32459261 | T/C | BTNL2 | 0.252 | 0.441 | 1.95E-08 | 0.43(0.31-0.58) |
| rs4424066 | 6 | 32462406 | A/G | BTNL2 | 0.252 | 0.44 | 2.49E-08 | 0.43(0.32-0.58) |
| rs9268472 | 6 | 32463583 | G/A | BTNL2 | 0.252 | 0.442 | 1.87E-08 | 0.43(0.31-0.58) |
| rs3117099 | 6 | 32466248 | G/A | BTNL2 | 0.728 | 0.462 | 6.07E-15 | 3.12(2.31-4.21) |
| rs3817973 | 6 | 32469089 | C/T | BTNL2 | 0.252 | 0.439 | 2.94E-08 | 0.43(0.32-0.58) |
| rs2076529 | 6 | 32471933 | T/C | BTNL2 | 0.252 | 0.442 | 1.85E-08 | 0.43(0.31-0.58) |
| rs4248166 | 6 | 32474399 | T/C | BTNL2 | 0.457 | 0.236 | 8.13E-14 | 2.71(2.07-3.56) |

(continued)

| rs number | chromosome | position | allele (A1/A2) | gene | integrated analysis | | | |
|---|---|---|---|---|---|---|---|---|
| | | | | | A2 frequency | | p value | OR (95%CI) |
| | | | | | patients | controls | | |
| rs2294884 | 6 | 32475237 | T/G | BTNL2 | 0.474 | 0.24 | 3.06E-15 | 2.85(2.17-3.73) |
| rs2294878 | 6 | 32475773 | G/T | BTNL2 | 0.517 | 0.322 | 1.05E-09 | 2.26(1.73-2.95) |
| rs3763304 | 6 | 32477333 | C/T | BTNL2 | 0.474 | 0.24 | 2.78E-15 | 2.85(2.18-3.74) |
| rs28362681 | 6 | 32478857 | C/T | BTNL2 | 0.457 | 0.159 | 2.60E-30 | 4.44(3.37-5.84) |
| rs28362683 | 6 | 32480941 | G/A | BTNL2 | 0.457 | 0.161 | 1.03E-29 | 4.37(3.32-5.75) |
| rs10947261 | 6 | 32481210 | G/T | BTNL2 | 0.596 | 0.405 | 1.23E-08 | 2.17(1.65-2.86) |
| rs10947262 | 6 | 32481290 | C/T | BTNL2 | 0.596 | 0.404 | 1.08E-08 | 2.18(1.66-2.85) |
| rs3806157 | 6 | 32481779 | T/G | BTNL2 | 0.5 | 0.242 | 4.83E-18 | 3.13(2.39-4.1) |
| rs3763313 | 6 | 32484449 | A/C | BTNL2 | 0.443 | 0.207 | 5.60E-17 | 3.06(2.33-4.02) |
| rs4959028 | 6 | 32491116 | A/G | BTNL2 | 0.439 | 0.15 | 3.55E-30 | 4.45(3.38-5.87) |
| snp15 | 6 | 32496295 | A/G | LOC646668 | 0.327 | 0.105 | 7.10E-24 | 4.17(3.09-5.61) |
| rs9268557 | 6 | 32497283 | T/C | LOC646668 | 0.241 | 0.506 | 1.46E-14 | 0.31(0.23-0.42) |
| rs3135363 | 6 | 32497626 | A/G | LOC646668 | 0.478 | 0.273 | 2.83E-11 | 2.44(1.86-3.19) |
| rs6930615 | 6 | 32500183 | G/A | LOC646668 | 0.259 | 0.078 | 9.19E-20 | 4.12(2.97-5.72) |

[Table 5]

| rs number | chromosome | position | allele (A1/A2) | gene | integrated analysis | | | |
|---|---|---|---|---|---|---|---|---|
| | | | | | A2 frequency | | p value | OR (95%CI) |
| | | | | | patients | controls | | |
| rs6457580 | 6 | 32501119 | G/T | LOC646668 | 0.304 | 0.079 | 2.00E-30 . | 5.12(3.77-6.95) |
| rs732162 | 6 | 32502891 | G/A | LOC646668 | 0.82 | 0.601 | 4.28E-11 | 3.03(2.15-4.27) |
| rs9501626 | 6 | 32508322 | C/A | HLA-DRA | 0.241 | 0.11 | 2.24E-09 | 2.58 (1.87-3.56) |
| snp16 | 6 | 32510683 | G/A | HLA-DRA | 0.241 | 0.113 | 7.27E-09 | 2.5(1.82-3.45) |
| rs3135392 | 6 | 32517220 | C/A | HLA-DRA | 0.57 | 0.36 | 2.03E-10 | 2.35(1.79-3.08) |
| rs8084 | 6 | 32519013 | A/C | HLA-DRA | 0.374 | 0.587 | 2.50E-10 | 0.42(0.32-0.55) |
| rs2239806 | 6 | 32519285 | C/T | HLA-DRA | 0.413 | 0.218 | 8.83E-12 | 2.53(1.92-3.33) |
| rs7192 | 6 | 32519624 | T/G | HLA-DRA | 0.374 | 0.587 | 2.27E-10 | 0.42(0.32-0.55) |
| rs3129888 | 6 | 32519704 | G/A | HLA-DRA | 0.868 | 0.957 | 1.25E-09 | 0.29(0.19-0.45) |
| rs7195 | 6 | 32520517 | A/G | HLA-DRA | 0.374 | 0.588 | 2.09E-10 | 0.42(0.32-0.55) |
| rs7197 | 6 | 32520558 | T/C | HLA-DRA | 0.883 | 0.966 | 2.34E-10 | 0.26(0.17-0.41) |
| rs1051336 | 6 | 32520570 | G/A | HLA-DRA | 0.413 | 0.22 | 1.56E-11 | 2.5(1.9-3.29) |
| rs1041885 | 6 | 32520787 | T/A | HLA-DRA | 0.413 | 0.22 | 1.68E-11 | 2.5(1.9-3.28) |
| rs2213586 | 6 | 32521072 | A/G | HLA-DRA | 0.374 | 0.588 | 2.09E-10 | 0.42(0.32-0.55) |
| rs2213585 | 6 | 32521128 | G/A | HLA-DRA | 0.374 | 0.587 | 2.19E-10 | 0.42(0.32-0.55) |
| rs2227139 | 6 | 32521437 | G/A | HLA-DRA | 0.374 | 0.588 | 2.08E-10 | 0.42(0.32-0.55) |
| rs3129890 | 6 | 32522251 | T/C | HLA-DRA | 0.635 | 0.426 | 5.69E-10 | 2.35(1.78-3.09) |
| snp17 | 6 | 32522265 | T/C | HLA-DRA | 0.232 | 0.504 | 1.73E-15 | 0.3(0.22-0.41) |
| rs3135387 | 6 | 32523087 | T/G | HLA-DRA | 0.741 | 0.886 | 1.20E-10 | 0.37(0.27-0.51) |
| rs9268681 | 6 | 32523364 | G/A | HLA-DRA | 0.211 | 0.419 | 5.54E-10 | 0.37(0.27-0.51) |
| rs6937545 | 6 | 32526009 | A/C | HLA-DRA | 0.443 | 0.697 | 1.24E-15 | 0.35(0.26-0.45) |
| rs12524661 | 6 | 32527182 | G/A | HLA-DRA | 0.13 | 0.044 | 4.10E-09 | 3.26(2.15-4.94) |
| rs7754768 | 6 | 32528157 | C/T | HLA-DRA | 0.235 | 0.505 | 1.77E-15 | 0.3(0.22-0.41) |
| rs7763262 | 6 | 32532860 | T/C | HLA-DRB9 | 0.443 | 0.697 | 1.22E-15 | 0.34(0.26-0.45) |
| rs9268832 | 6 | 32535767 | T/C | HLA-DRB9 | 0.259 | 0.589 | 1.42E-22 | 0.24(0.18-0.33) |
| rs6903608 | 6 | 32536263 | C/T | HLA-DRB9 | 0.457 | 0.754 | 4.34E-23 | 0.27(0.21-0.36) |
| snp18 | 6 | 32537572 | A/G | HLA-DRB9 | 0.491 | 0.683 | 4.60E-09 | 0.45(0.34-0.59) |
| rs9268877 | 6 | 32539125 | A/G | HLA-DRB9 | 0.487 | 0.68 | 1.88E-09 | 0.45(0.34-0.59) |
| rs9268880 | 6 | 32539336 | T/G | HLA-DRB9 | 0.509 | 0.762 | 1.23E-17 | 0.32(0.25-0.42) |
| snp19 | 6 | 32539466 | C/A | HLA-DRB9 | 0.117 | 0.033 | 5.55E-11 | 3.95(2.54-6.15) |

(continued)

| rs number | chromosome | position | allele (A1/A2) | gene | integrated analysis | | | |
|---|---|---|---|---|---|---|---|---|
| | | | | | A2 frequency | | p value | OR (95%CI) |
| | | | | | patients | controls | | |
| rs9268979 | 6 | 32543022 | T/C | HLA-DRB9 | 0.491 | 0.677 | 1.15E-08 | 0.46(0.35-0.6) |
| rs9269110 | 6 | 32551247 | A/C | HLA-DRB9 | 0.509 | 0.76 | 2.44E-17 | 0.33(0.25-0.43) |
| rs1964995 | 6 | 32557389 | T/C | HLA-DRB9 | 0.196 | 0.428 | 4.05E-12 | 0.33(0.23-0.45) |
| rs4713555 | 6 | 32683502 | G/T | HLA-DRB1 | 0.448 | 0.232 | 1.56E-13 | 2.69(2.05-3.52) |
| rs9271586 | 6 | 32698877 | G/T | HLA-DQA1 | 0.261 | 0.5 | 2.00E-12 | 0.35(0.26-0.48) |
| rs9271588 | 6 | 32698931 | T/C | HLA-DQA1 | 0.259 | 0.5 | 1.58E-12 | 0.35(0.26-0.47) |
| rs9271667 | 6 | 32700688 | T/C | HLA-DQA1 | 0.422 | 0.248 | 5.85E-09 | 2.21(1.68-2.9) |
| rs9271850 | 6 | 32703038 | A/G | HLA-DQA1 | 0.46 | 0.26 | 5.26E-11 | 2.43(1.85-3.19) |
| rs7744001 | 6 | 32734064 | G/A | LOC646686 | 0.548 | 0.318 | 8.93E-13 | 2.59(1.98-3.39) |
| rs6689 | 6 | 32735678 | A/G | HLA-DQB1 | 0.074 | 0.236 | 1.21E-08 | 0.26(0.16-0.43) |
| snp20 | 6 | 32778086 | C/T | HLA-DQB1 | 0.066 | 0.015 | 3.11E-08 | 4.56(2.53-8.2) |
| snp21 | 6 | 32790340 | T/C | HLA-DQA2 | 0.265 | 0.076 | 1.08E-22 | 4.36(3.17-5.99) |
| rs9275659 | 6 | 32794081 | C/A | HLA-DQA2 | 0.263 | 0.084 | 4.17E-19 | 3.88(2.82-5.33) |

[Table 6]

| rs number | chromosome | position | allele (A1/A2) | gene | integrated analysis | | | |
|---|---|---|---|---|---|---|---|---|
| | | | | | A2 frequency | | p value | OR (95%CI) |
| | | | | | patients | controls | | |
| rs9275682 | 6 | 32795275 | A/G | HLA-DQA2 | 0.265 | 0.087 | 1.09E-18 | 3.8(2.77-5.22) |
| rs9275686 | 6 | 32795548 | G/A | HLA-DQA2 | 0.263 | 0.084 | 4.53E-19 | 3.87(2.82-5.32) |
| rs763026 | 6 | 32799723 | C/T | HLA-DQA2 | 0.265 | 0.083 | 3.17E-20 | 4.01(2.92-5.5) |
| rs9276171 | 6 | 32806896 | A/G | HLA-DQA2 | 0.283 | 0.102 | 4.35E-17 | 3.48(2.56-4.73) |
| snp22 | 6 | 32825993 | G/T | HLA-DQA2 | 0.237 | 0.07 | 4.78E-19 | 4.11(2.94-5.74) |
| rs2621358 | 6 | 32878786 | C/T | HLA-DOB | 0.513 | 0.334 | 3.21E-08 | 2.1(1.61-2.74) |
| rs2621338 | 6 | 32884561 | G/A | HLA-DOB | 0.513 | 0.333 | 2.79E-08 | 2.11(1.61-2.75) |
| rs2857118 | 6 | 32885878 | G/A | HLA-DOB | 0.513 | 0.333 | 2.58E-08 | 2.11(1.61-2.76) |
| rs2857115 | 6 | 32886259 | G/A | HLA-DOB | 0.513 | 0.333 | 2.31E-08 | 2.11(1.62-2.76) |
| snp23 | 6 | 32887918 | T/A | HLA-DOB | 0.513 | 0.333 | 2.78E-08 | 2.11(1.61-2.76) |
| rs2228391 | 6 | 32905751 | T/C | TAP2 | 0.243 | 0.067 | 2.62E-22 | 4.5(3.24-6.25) |
| rs3819721 | 6 | 32912776 | G/A | TAP2 | 0.374 | 0.211 | 8.43E-09 | 2.23(1.69-2.95) |
| rs6906708 | 6 | 32976762 | G/T | PPP1R2P1 | 0.113 | 0.034 | 1.71E-09 | 3.62(2.32-5.66) |
| snp24 | 6 | 32983891 | G/A | HLA-DMB | 0.113 | 0.032 | 1.89E-10 | 3.89(2.49-6.1) |

The 191 SNPs showing extremely strong association with antithyroid drug-induced agranulocytosis were largely divided into total 4 regions of 2 regions in Class I region (upstream side of HLA-A region and around HLA-B region) and 2 regions in Class II region (2 regions around HLA-DR region) (Fig. 2). The strongest association was obtained around HLA-DR region (most strongly associated polymorphism: rs6457580, $p=2.0x10^{-30}$, OR:5.12 (95%CI:3.77-6.95)), then around HLA-B region (most strongly associated polymorphism: rs41560220, $p=2.1x10^{-26}$, OR:5.44 (95%CI:3.85-7.68)), HLA-A region upstream (most strongly associated polymorphism: rs1736959, $p=1.7x10^{-12}$, OR:2.79 (95%CI: 2.07-3.74)), around HLA-DR region (most strongly associated polymorphism: rs3135387, $p=1.2x10^{-10}$, OR: 0.37 (95%CI:0.27-0.51), then strongly associated polymorphism: rs17576984, $p=1.15x10^{-27}$, OR: 4.17 (95%CI:3.17-5.49)) (Table 7).

[Table 7]

| SNP | chromosome | position | allele (A1/A2) | neighboring gene | integrated analysis | | |
|---|---|---|---|---|---|---|---|
| | | | | | A2 frequency cases/controls | p value | OR (95%CI) |
| rs6457580 | 6 | 32393141 | G/T* | LOC646668 | 0.333/0.076 | $2.0 \times 10^{-30}$ | 5.12 (3.77-6.95) |
| rs41560220 | 6 | 31323875 | C/T* | HLA-B | 0.218/0.049 | $2.1 \times 10^{-26}$ | 5.44 (3.85-7.68) |
| rs1736959 | 6 | 29782470 | C/T* | HLA-G | 0.282/0.138 | $1.7 \times 10^{-12}$ | 2.79 (2.07-3.74) |
| rs3135387 | 6 | 32415109 | T*/G | HLA-DRA | 0.774/0.884 | $1.2 \times 10^{-10}$ | 0.37 (0.27-0.51) |
| rs17576984 | 6 | 32320963 | C/T* | LOC401252 | 0.456/0.167 | $1.15 \times 10^{-27}$ | 4.17 (3.17-5.49) |
| 1) A1 and A2 show reference allele and variant allele of NCBI GRCh37, and * shows risk allele. | | | | | | | |

It was found that many of the SNPs showing a significant difference in each region were in strong linkage disequilibrium (Figs. 3 - 5). The 4 regions did not show linkage disequilibrium with each other, and were found to be independent. While rs17576984 and rs3135387 were found in the same region, they showed linkage disequilibrium coefficient D'=0.44 and were independent.

To further verify whether SNP markers for antithyroid drug-induced agranulocytosis were independent of each other, multiple logistic regression analysis of rs6457580, rs41560220, rs1736959 and rs3135387 was performed (Table 8).

[Table 8]

| logistic regression analysis | | | | | |
|---|---|---|---|---|---|
| SNP | chromosome | position | allele (A1/A2) | p value | OR (95%CI) |
| rs6457580 | 6 | 32393141 | G/T* | $3.4 \times 10^{-23}$ | 7.87 (5.19-11.93) |
| rs41560220 | 6 | 31323875 | C/T* | $6.0 \times 10^{-10}$ | 3.95 (2.54-6.16) |
| rs1736959 | 6 | 29782470 | C/T* | $5.3 \times 10^{-12}$ | 3.51 (2.44-5.06) |
| rs3135387 | 6 | 32415109 | T*/G | $3.1 \times 10^{-9}$ | 0.3 (0.20-0.45) |
| 1) A1 and A2 show reference allele and variant allele of NCBI GRCh37, and * shows risk allele. | | | | | |

As a result of multiple logistic regression analysis, all 4 markers independently showed significant associations with agranulocytosis.

Sequentially, whether the risk of antithyroid drug-induced agranulocytosis increases when the patients have plural risk alleles was examined. For the above-mentioned 4 SNPs, a target having no risk allele was used as reference. As a result, a rapid increase in the risk of antithyroid drug-induced agranulocytosis agranulocytosis was observed depending on the number of risk alleles of the patients. The odds ratio of the patients having all 4 risk alleles reached 953.2 (95%CI: 101.1-8988.6) (Fig. 6).

Furthermore, to avoid agranulocytosis, the usefulness of genetic screening before antithyroid drug therapy was also evaluated. For this end, a type condition risk of developing the complication was calculated based on the distribution of risk alleles of 4 SNP markers in the patients and the control group. When the morbidity rate of antithyroid drug-induced agranulocytosis is 0.35% (reference), 38.3% of patients having 4 risk alleles were estimated to develop agranulocytosis (Table 9). the morbidity rate of agranulocytosis of patients having 3 risk alleles was assumed to be 3.8%.

[Table 9]

| number of risk allele | case | | control | | OR (95% CI) | type condition risk of agranulocytosis |
|---|---|---|---|---|---|---|
| | number (ratio) | cumulative number (ratio) | number (ratio) | cumulative number (ratio) | | |
| 4 | 7 (0.061) | 7 (0.061) | 1 (0.001) | 1 (0.001) | 953.17 (101.08-8988.59) | 0.383 |
| 3 | 22 (0.191) | 29 (0.252) | 32 (0.018) | 33 (0.018) | 93.61 (35.51-246.78) | 0.038 |
| 2 | 49 (0.426) | 78 (0.678) | 246 (0.137) | 279 (0.155) | 27.12 (11.48-64.07) | 0.011 |
| 1 | 31 (0.270) | 109 (0.948) | 702 (0.390) | 981 (0.546) | 6.01 (2.49-14.50) | 0.0024 |
| 0 | 6 (0.052) | 115 (1.000) | 817 (0.454) | 1798 (1.000) | Reference | 0.00040 |

Of the patients of 115 cases used for this study, 113 cases had Graves' disease. Thus, the association of agranulocytosis and the above-mentioned SNP is caused by Graves' disease itself was examined. To be specific, DNAs derived from 89 Graves' disease patients were directly sequenced for genotyping of 4 SNP markers (rs6457580, rs41560220, rs1736959 and rs3135387), and compared with the frequency in the control group. As a result, none of these markers showed association with Graves' disease.

[Example 2]

Search for HLA allele correlated to antithyroid drug-induced agranulocytosis

(1) HLA genotyping

Since all the SNP markers obtained above were present in the HLA region, there is a possibility that antithyroid drug-induced agranulocytosis is related to the HLA gene per se. Thus, genotyping of HLA-B, HLA-C, HLA-DRB1, HLA-DPB1 and HLA-DQB1 genes was performed using the patient samples while targeting around the HLA-DR region and HLA-B region showing extremely strong association.

To be specific, the genotypes of HLA-B, HLA-C, HLA-DRB1, HLA-DPB1 and HLA-DQB1 genes of 115 cases of antithyroid drug-induced agranulocytosis patients described in Example 1 were determined by high resolution (4-digit) genotyping using WAKFlow system (Wakunaga Pharmaceutical Co., Ltd, Osaka, Japan). HLA-B, HLA-C, HLA-DRB1, HLA-DPB1 and HLA-DQB1 allele frequency information of a population of 1,000 general Japanese people was obtained from a non-profit organization, HLA Laboratory (Kyoto, Japan). In HLA genotyping, HLA allele having an allele frequency exceeding 1% was used for the association study in the case group or control group.

In statistical analysis, the HLA allele frequencies were compared between the case group and the control group by the chi-squared test or Fisher's exact probability test. Multiple logistic regression analysis was performed to verify independence of HLA alleles. The significant level after Bonferroni's correction in the multiple test was set to $6.9x10^{-4}$.

(2) results

After Bonferroni's correction in the multiple test, HLA-B*39:01, HLA-B*38:02, HLA-C*07:02, HLA-DRB1*08:03, HLA-DRB1*14:03, HLA-DRB1*08:02, HLA-DRB1*09:01 and HLA-DQB1*06:01 showed significant association with antithyroid drug-induced agranulocytosis (Table 10).

[Table 10]

| association of agranulocytosis and HLA allele | | | | | | |
|---|---|---|---|---|---|---|
| HLA allele | chromosome number | | association | | multiple logistic regression analysis | |
| | case (n=230) | control (n=2,000) | p value | OR (95%CI) | p value | OR (95%CI) |
| B*38:02 | 5 | 1 | $6.2 \times 10^{-5}$ | 44.42 (5.17-381.91) | 0.0033 | 32.64 (3.04-350.48) |
| B*39:01 | 39 | 58 | $4.3 \times 10^{-23}$ | 6.84 (4.44-10.53) | $8.7 \times 10^{-9}$ | 4.51 (2.67-7.61) |
| C*07:02 | 59 | 231 | $1.7 \times 10^{-9}$ | 2.64 (1.91-3.66) | N.S. | - |
| DRB1*08:02 | 26 | 91 | $1.4 \times 10^{-5}$ | 2.67 (1.69-4.23) | $4.3 \times 10^{-6}$ | 3.55 (2.05-6.16) |
| DRB1*08:03 | 70 | 164 | $2.0 \times 10^{-25}$ | 4.90 (3.55-6.77) | $4.8 \times 10^{-14}$ | 4.57 (3.06-6.85) |
| DRB1*09:01 | 16 | 309 | $5.5 \times 10^{-4}$ | 0.41 (0.24-0.69) | N.S. | - |
| DRB1*14:03 | 12 | 24 | $4.7 \times 10^{-6}$ | 4.53 (2.24-9.19) | $7.0 \times 10^{-7}$ | 7.05 (3.21-15.48) |
| DQB1*06:01 | 81 | 390 | $3.2 \times 10^{-8}$ | 2.24 (1.68-3.01) | N.S. | - |
| 1) N.S. shows not significant in multiple logistic regression analysis using HLA allele. | | | | | | |

Of the above-mentioned 8 HLA alleles, HLA-B*39:01 was in linkage disequilibrium (LD) (D'=0.97) with HLA-C*07:02, and HLA-DRB1*08:03 was in LD (D'=0.96) with HLA-DQB1*06:01. Multiple logistic regression analysis clarified significant and independent associations of HLA-B*39:01, HLA-DRB1*08:03, HLA-DRB1*14:03 and HLA-DRB1*08:02 with agranulocytosis, and detected weak association (p=0.0033) with HLA-B*38:02.

Whether the clinical progress of agranulocytosis is influenced by the above-mentioned SNP markers in significant association with agranulocytosis, and HLA alleles identified in the patients was examined by linear regression analysis. The above-mentioned SNP markers and HLA allele showed no association with any of the age of diagnosis, the period from the start of an antithyroid drug to the diagnosis with agranulocytosis. In addition, the allele frequencies of these SNP markers and HLA alleles were not different between 95 patients treated with methimazole and 20 patients treated with PTU, ($p \geq 0.011$).

[Example 3]

Search for amino acid in association with susceptibility HLA allele

(1) logistic regression analysis of HLA amino acid

An amino acid sequence corresponding to either one of HLA alleles, which was genotyped or obtained from HLA Laboratory, was searched for in the IMGT database (http://www.ebi.ac.uk/ipd/imgt/hla/), and aligned in each HLA gene. A total of 462 amino acid variants were identified in 278 sites. Three-dimensional structural analysis of HLA-DRB1 and HLA-B protein was performed using UCSF chimera software.

(2) set up multiple logistic regression analysis

Since plural alleles showed association with agranulocytosis in both HLA-B and HLA-DRB1 genes, the presence of an important amino acid residue in susceptibility HLA alleles was assumed. Therefore, set up multiple logistic regression analysis was performed using the alignment of HLA amino acid sequences.

(3) statistical analysis

Akaike Information Criterion (AIC) was calculated for the amino acid analysis. When a logistic regression model containing mutation shows the smallest AIC, amino acid mutation was considered significant than other mutation when AIC is larger than 7 as compared to that of other amino acid mutation (ΔAIC>7). The permutation test was performed 1,000 times, and whether improvement of AIC by amino acid mutation was accidentally afforded, and whether improvement of AIC by significant amino acid mutation is equivalent to that by a significant SNP marker were evaluated.

(4) results

Position 74 leucine residue of HLA-DRB1 protein (DRB1-Leu74) showed the strongest association of ΔAIC 21.0 as compared to other amino acid mutations ($p=7.9x10^{-26}$, permutation p=0.001, Fig. 7A). Following the conditioning of DRB1-Leu74, position 116 phenylalanine (B-Phe116) showed second strongest association of ΔAIC 14.1 ($p<8.2x10^{-12}$, permutation p=0.001, Fig. 7B). The absence of the alanine residue at position 158 of HLA-B protein was found to be in linkage disequilibrium ($r^2=0.92$) with the presence of B-Phe116. After the conditioning of DRB1-Leu74 and B-Phe116, other amino acids did not show significant association (p>0.00024, Fig. 7C). Importantly, HLA-B*39:01 and HLA-B*38:02 had B-Phe116, and HLA-DRB1*08:03, HLA-DRB1*14:03 and HLA-DRB1*08:02 had DRB1-Leu74 (Table 11). rs41560220 of HLA-B and rs6457580 and rs3135387 of HLA-DRB1 showed equivalent improvement of AIC as compared to B-Phe116 and DRB1-Leu74, respectively.

[Table 11]

| | frequency case/control | before adjustment | | after adjustment | | HLA allele |
|---|---|---|---|---|---|---|
| | | p value | OR (95%CI) | p value | OR (95%CI) | |
| **Class I B 116 position** | | | | | | |
| Phe | 0.23/0.05 | $1.2x10^{-24}$ | 5.89 (3.94-8.21) | $8.1x10^{-12}$ | 4.59 (2.97-7.11) | *37:01, *38:02, *39:01, *39:04, *67:01 |
| Asp. Ala, Ser, Leu, Tyr | 0.77/0.95 | - | **reference** | - | **reference** | *07:02, *13:01, *15:01, *15:07, *15:11, *15:18, *27:05, *35:01, *40:01, *40:02, *40:06, *44:03, *46:01, *48:01, *51:01, *52:01, *54:01, *55:02, *58:01, *59:01 |
| **Class II DRB1 74 position** | | | | | | |
| Leu | 0.47/0.14 | $8.8x10^{-37}$ | 5.56 (4.17-7.41) | $1.8x10^{-20}$ | 5.00 (3.56-7.03) | *08:02, *08:03, *08:09, *14:03 |
| Ala, Glu, Gln | 0.53/0.86 | - | **reference** | - | **reference** | *01:01, *03:01, *04:03, *04:05, *04:06, *04:10, *09:01, *10:01, *11:01, *12:01, *12:02, *13:02, *14:05, *14:06, *14:07, *14:54, *15:01, *15:02, *16:02 |

[Example 4]

<u>odds ratio for each haplotype with respect to the SNP markers of the present invention and simulation thereof</u>

Using the samples of 115 patients with agranulocytosis caused by antithyroid drugs and 1937 healthy individuals, the odds ratios (ORs, for each haplotype) of the SNP markers of the present invention rs2596487, which is in complete linkage D'=1 with rs41560220, and rs17576984 were analyzed by association study for each SNP. When the both SNPs had a risk allele, OR of multiple logistic regression analysis was applied. Based on the results of allele frequencies of the SNPs in 1937 healthy individual samples, and supposing that the Hardy-Weinberg equilibrium stands, the ratio of each haplotype frequency in a general healthy individual population was calculated. From the haplotype frequencies and the above-mentioned ORs in healthy individuals, moreover, the ratio of involvement of the haplotypes in the onset was analyzed. The results are shown in the following Table 12. In Table 12, homozygote for risk allele is shown as score 2, heterozygote for risk allele and non-risk allele is shown as score 1, and homozygote for non-risk allele is shown as score 0. When SNP does not have a risk allele, it is used as the control.

[Table 12]

| | AF | OR | geno00 | geno01 | geno10 | geno02 | geno20 | geno11 | geno12 | geno21 | geno22 |
|---|---|---|---|---|---|---|---|---|---|---|---|
| rs2596487 | 0.96 | 6 | 0 | 0 | 1 | 0 | 2 | 1 | 1 | 2 | 2 |
| rs17576984 | 0.84 | 4.5 | 0 | 1 | 0 | 2 | 0 | 1 | 2 | 1 | 2 |
| | | OR | 1 | 4.5 | 6 | 20.25 | 36 | 19.95 | 75.8 | 104.7 | 398.0 |
| haplotype frequency | | | 0.65 | 0.248 | 0.054 | 0.024 | 0.0011 | 0.0206 | 0.00197 | 0.00043 | 0.000041 |
| rate of involvement in onset | | | 0.186 | 0.318 | 0.093 | 0.136 | 0.012 | 0.118 | 0.0425 | 0.013 | 0.0047 |

As shown in Table 12, when two SNPs of rs2596487 and rs17576984 were tested, the scores of rs2596487/rs17576984 showed an increasing odds ratio in the order of 0/0, 0/1, 1/0, 0/2, 2/0, 1/1, 1/2, 2/1, 2/2 genotypes, and the odds ratio of patients homozygous for the risk alleles for both SNPs reached 398.0. From the genotype ratio of SNP of antithyroid drug-induced agranulocytosis patients, and the rate of involvement of the genotype in the onset, it is possible to predict how far the onset of agranulocytosis can be avoided by the discontinuation of antithyroid drug administration when patients have what genotype (Table 13). To be specific, as shown in Table 13, for example, when patients having a genotype after geno01 (i.e., geno01, 10, 02, 20, 11, 12, 21, 22) discontinue antithyroid drug administration, it means that 35% of patients discontinue antithyroid drug administration, which reduces the number of patients who develop agranulocytosis by 81%. When patients having a genotype after geno10 (i.e., geno10, 02, 20, 11, 12, 21, 22) discontinue antithyroid drug administration, it means that 10% of patients discontinue antithyroid drug administration, which reduces the number of patients who develop agranulocytosis by 50%. Whether antithyroid drug administration should be discontinued when the patient has what genotype can be appropriately determined by the judgment of medical doctors, in consideration of various factors such as severity of disease and complication thereof (particularly, kidney hypofunction), age of patient (elderly people), sex and the like.

[Table 13]

| | not discontinued | discontinued after geno01 | discontinued after geno10 | discontinued after geno02 |
|---|---|---|---|---|
| percent discontinuation | 0 | 35% | 10% | 4.8% |
| number of onset of agranulocytosis | 100% | 81% decrease | 50% decrease | 40% decrease |

Industrial Applicability

According to the present invention, the risk of antithyroid drug-induced agranulocytosis can be determined conveniently and highly accurately. Therefore, in patients determined to have a high risk of antithyroid drug-induced agranulocytosis, the onset of extremely serious side effects can be avoided by selecting a treatment method other than a drug therapy in treating hyperthyroidism and, in patients determined to have a low risk thereof, invasive tests such as excessive blood sampling and the like can be avoided. As a result, a safe, secure and accurate treatment of hyperthyroidism becomes possible.

This application is based on patent application No. 2013-188806 filed in Japan (filing date: September 11, 2013), the contents of which are incorporated in full herein.

## Claims

1. A test method for determining the risk of antithyroid drug-induced agranulocytosis, comprising

    (1) a step of using a sample derived from a test subject and testing polymorphism present in the HLA region, which is at least one selected from the group consisting of

        A) polymorphism at the 501st nucleotide in the nucleotide sequence shown in SEQ ID NO: 1 (G>T*),
        B) polymorphism at the 201st nucleotide in the nucleotide sequence shown in SEQ ID NO: 2 (C>T*),
        C) polymorphism at the 501st nucleotide in the nucleotide sequence shown in SEQ ID NO: 3 (C>T*),
        D) polymorphism at the 501st nucleotide in the nucleotide sequence shown in SEQ ID NO: 4 (T*>G),
        E) polymorphism at the 501st nucleotide in the nucleotide sequence shown in SEQ ID NO: 5 (C>T*),
        wherein parentheses show reference allele>variant allele, * is risk allele, G, A, T and C are guanine, adenine, thymine and cytosine, respectively, and
        F) polymorphism in linkage disequilibrium with the polymorphism of any of the above-mentioned A) - E), the linkage disequilibrium showing a linkage disequilibrium coefficient D' of not less than 0.8, and

    (2) a step of determining the risk of antithyroid drug-induced agranulocytosis based on the test results of (1).

2. The test method according to claim 1, comprising a step of testing the polymorphism of the above-mentioned A) or polymorphism in linkage disequilibrium with said polymorphism at a linkage disequilibrium coefficient D' of not less than 0.8, and/or the polymorphism of B) or polymorphism in linkage disequilibrium with said polymorphism at a linkage disequilibrium coefficient D' of not less than 0.8.

3. The test method according to claim 2, wherein the polymorphism in linkage disequilibrium with the polymorphism of the above-mentioned A) at a linkage disequilibrium coefficient D' of not less than 0.8 is polymorphism at a position encoding the amino acid at position 74 of HLA-DRB1*08:03 or HLA-DRB1*08:02, and the polymorphism in linkage disequilibrium with the polymorphism of the above-mentioned B) at a linkage disequilibrium coefficient D' of not less than 0.8 is polymorphism at a position encoding the amino acid at position 116 or position 158 of HLA-B*39:01 or HLA-B*38:02.

4. The test method according to any one of claims 1 to 3, wherein the sample derived from the test subject contains genomic DNA.

5. The test method according to any one of claims 1 to 4, wherein the test subject is an eastern Asian.

6. A kit for determination of the risk of antithyroid drug-induced agranulocytosis, comprising a polynucleotide capable of detecting a risk allele in
polymorphism present in the HLA region, which is at least one selected from the group consisting of

    A) polymorphism at the 501st nucleotide in the nucleotide sequence shown in SEQ ID NO: 1 (G>T*),
    B) polymorphism at the 201st nucleotide in the nucleotide sequence shown in SEQ ID NO: 2 (C>T*),
    C) polymorphism at the 501st nucleotide in the nucleotide sequence shown in SEQ ID NO: 3 (C>T*),

D) polymorphism at the 501st nucleotide in the nucleotide sequence shown in SEQ ID NO: 4 (T*>G),
E) polymorphism at the 501st nucleotide in the nucleotide sequence shown in SEQ ID NO: 5 (C>T*),
wherein parentheses show reference allele>variant allele, * is risk allele, G, A, T and C are guanine, adenine, thymine and cytosine, respectively, and
F) polymorphism in linkage disequilibrium with the polymorphism of any of the above-mentioned A) - E), the linkage disequilibrium showing a linkage disequilibrium coefficient D' of not less than 0.8.

**7.** The kit according to claim 6, comprising a polynucleotide capable of detecting a risk allele of the polymorphism of the above-mentioned A) or polymorphism in linkage disequilibrium with said polymorphism at a linkage disequilibrium coefficient D' of not less than 0.8 and/or the polymorphism of B) or polymorphism in linkage disequilibrium with said polymorphism at a linkage disequilibrium coefficient D' of not less than 0.8.

**8.** The kit according to claim 7, wherein the polymorphism in linkage disequilibrium with the polymorphism of the above-mentioned A) at a linkage disequilibrium coefficient D' of not less than 0.8 is polymorphism at a position encoding the amino acid at position 74 of HLA-DRB1*08:03 or HLA-DRB1*08:02, and the polymorphism in linkage disequilibrium with the polymorphism of the above-mentioned B) at a linkage disequilibrium coefficient D' of not less than 0.8 is polymorphism at a position encoding the amino acid at position 116 or position 158 of HLA-B*39:01 or HLA-B*38:02.

**9.** The kit according to any one of claims 6 to 8, further comprising a polynucleotide capable of detecting a non-risk allele.

**10.** The kit according to any one of claims 6 to 9, wherein the above-mentioned polynucleotide capable of detecting a risk allele is a probe capable of hybridizing with a fragment of 10 - 200 continuous nucleotide sequence containing said allele or a complementary chain sequence thereof under stringent conditions, and/or a primer capable of amplifying said fragment.

**11.** The kit according to any one of claims 6 to 10, which is used for determining the risk of antithyroid drug-induced agranulocytosis in eastern Asians.

**12.** A test method for determining the risk of antithyroid drug-induced agranulocytosis, comprising

(1) a step of using a sample derived from a test subject and testing the following (a) and/or (b):

(a) whether the amino acid at position 74 of HLA-DRB1 protein is Leu
(b) whether the amino acid at position 116 of HLA-B protein is Phe, or the amino acid at position 158 of HLA-B protein is Ala, and

(2) a step of determining the risk of antithyroid drug-induced agranulocytosis based on the test results of (1).

**13.** A kit for determination of the risk of antithyroid drug-induced agranulocytosis, comprising a substance capable of identifying the following (a) and/or (b):

(a) the amino acid at position 74 of HLA-DRB1 protein is Leu
(b) the amino acid at position 116 of HLA-B protein is Phe, or the amino acid at position 158 of HLA-B protein is Ala.

Fig. 1

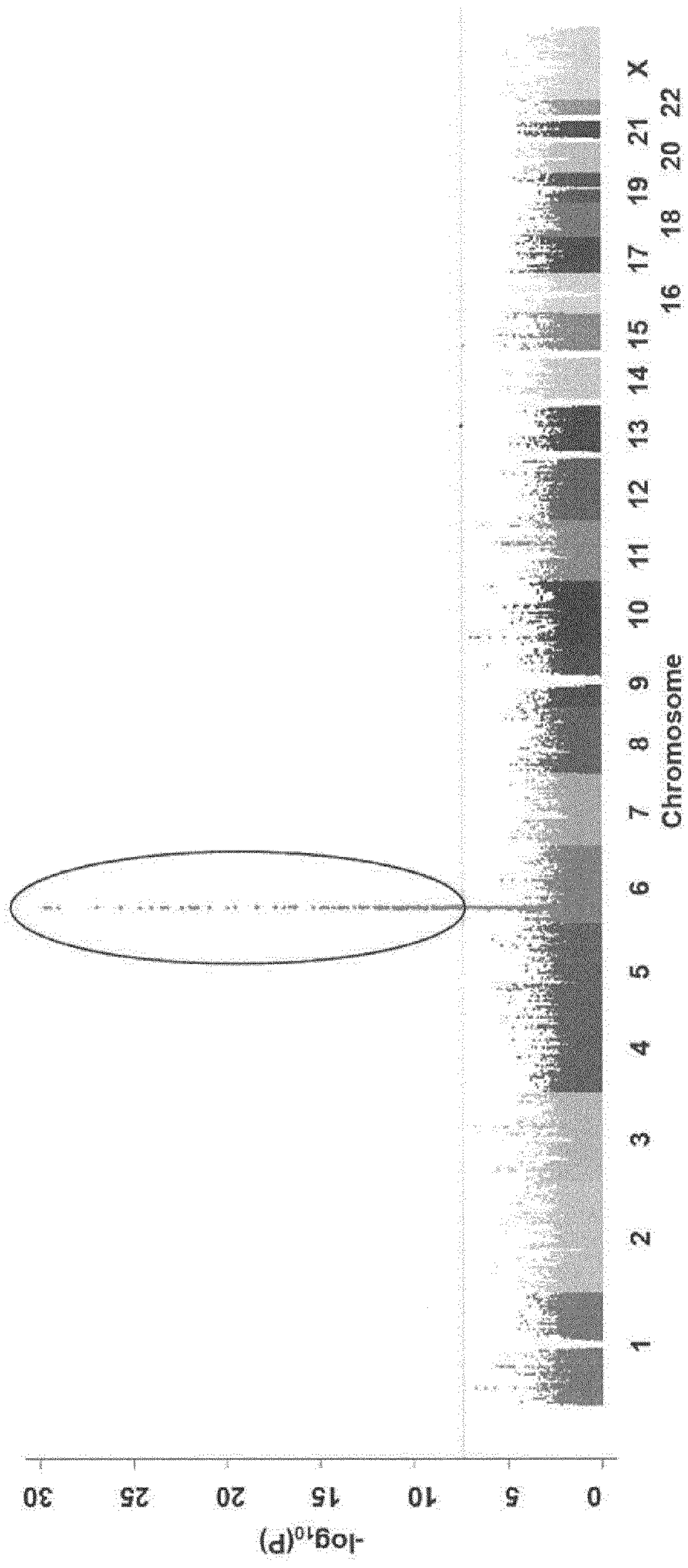
-log10(P)
0
5
10
15
20
25
30
Chromosome
1
2
3
4
5
6
7
8
9
10
11
12
13
14
15
16
17
18
19
20
21
22
X

Fig. 2

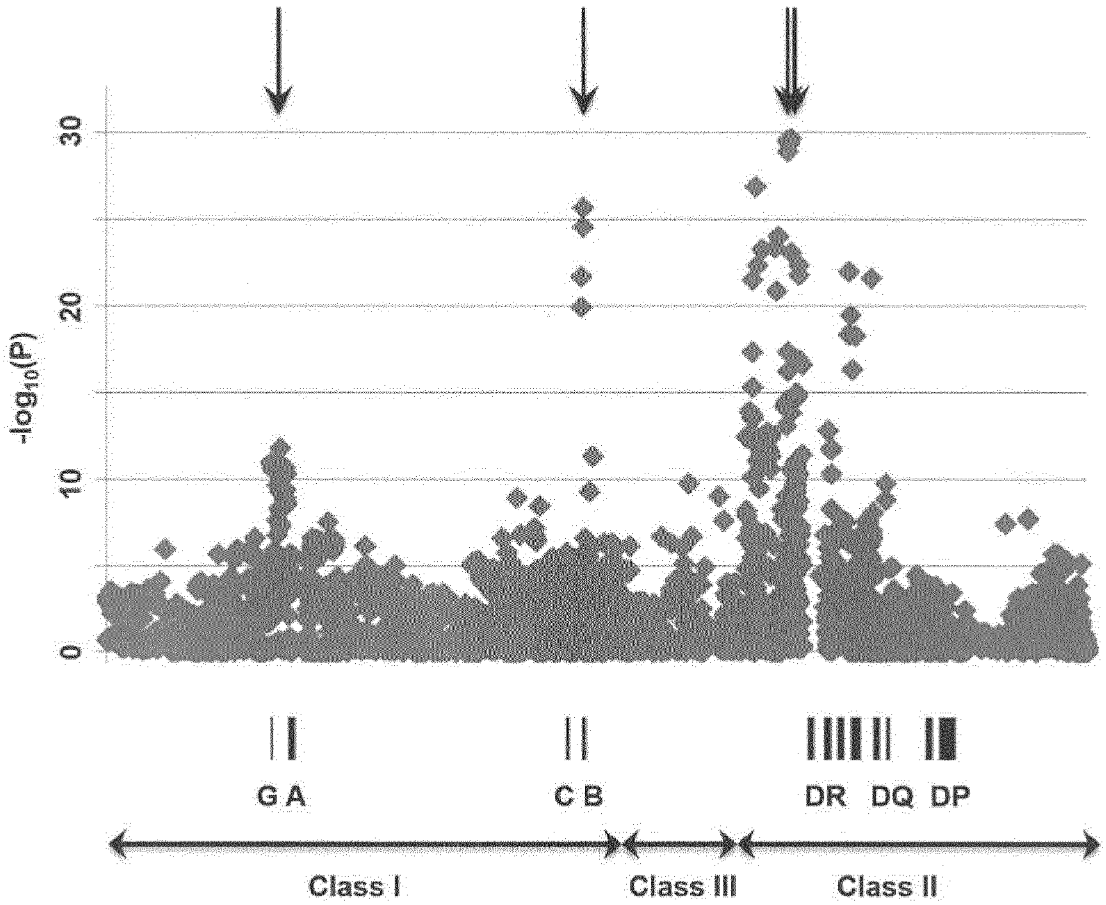

Fig. 3

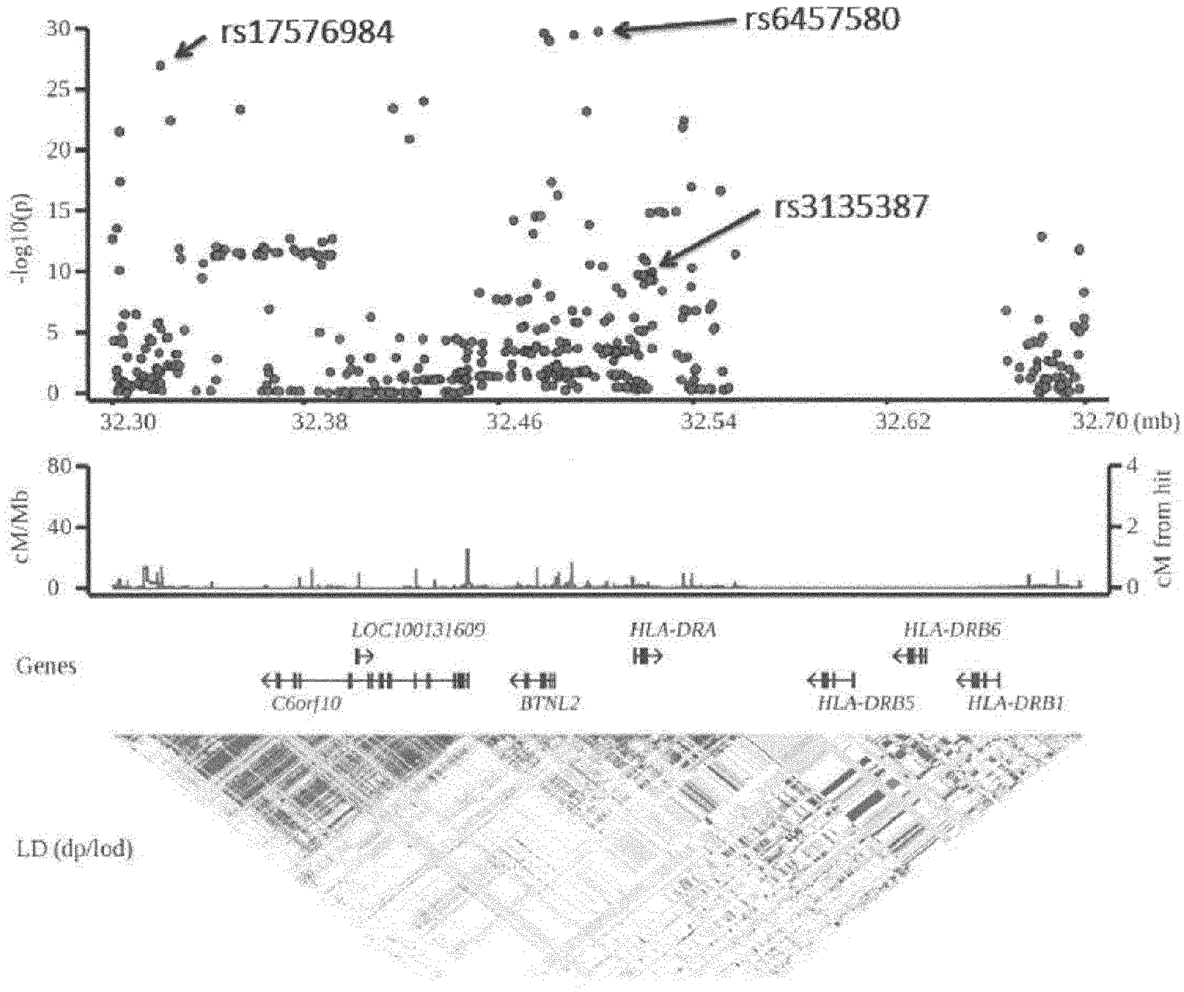
rs17576984
rs6457580
rs3135387
-log10(p)
30
25
20
15
10
5
0
32.30
32.38
32.46
32.54
32.62
32.70 (mb)
cM/Mb
80
40
0
cM from hit
4
2
0
Genes
LOC100131609
HLA-DRA
HLA-DRB6
C6orf10
BTNL2
HLA-DRB5
HLA-DRB1
LD (dp/lod)

Fig. 4

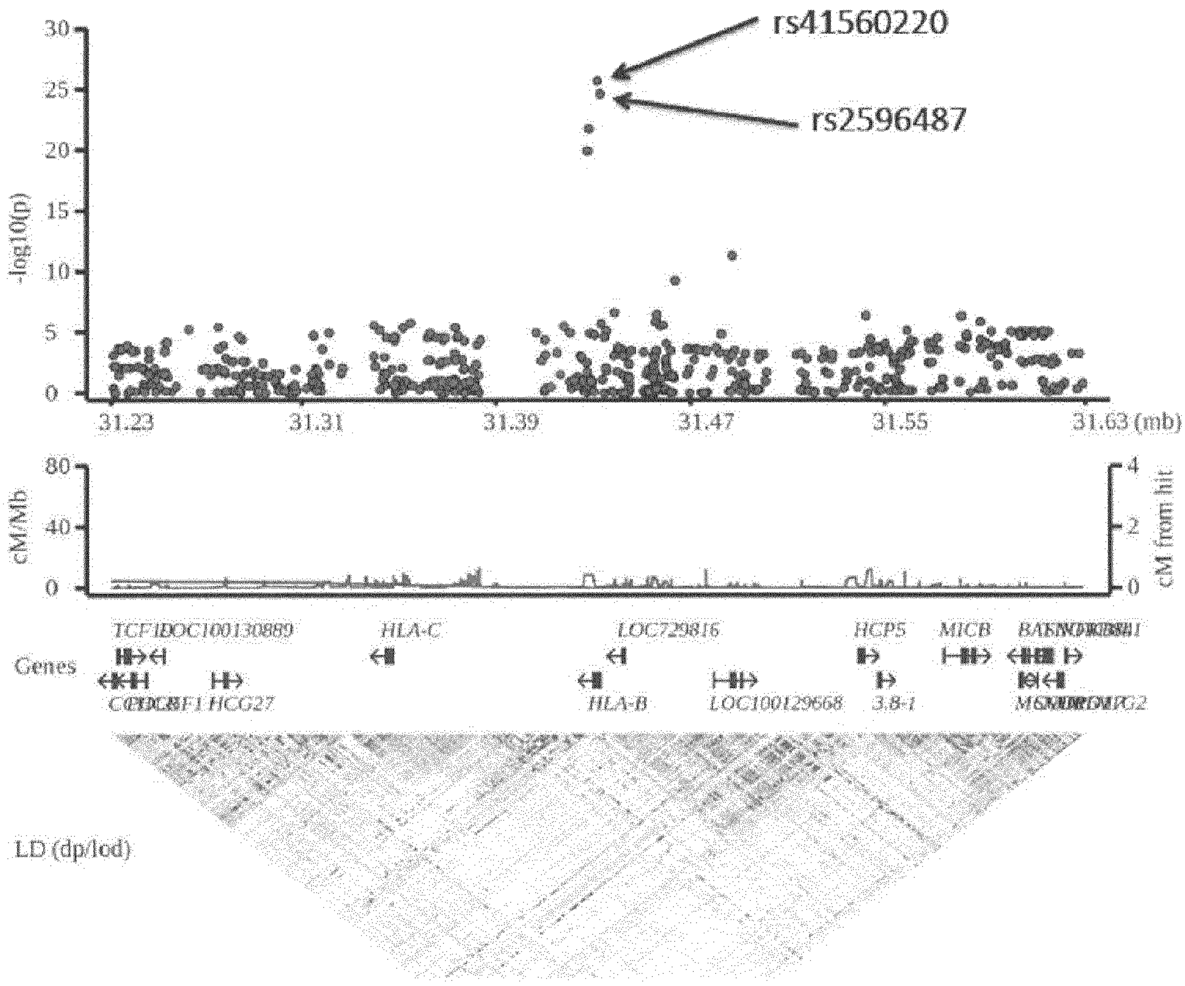
rs41560220
rs2596487
-log10(p)
30
25
20
15
10
5
0
31.23
31.31
31.39
31.47
31.55
31.63 (mb)
cM/Mb
80
40
0
cM from hit
4
2
0
Genes
HLA-C
LOC729816
HCP5
MICB
HCG27
HLA-B
LOC100129668
3.8-1
LD (dp/lod)

Fig. 5

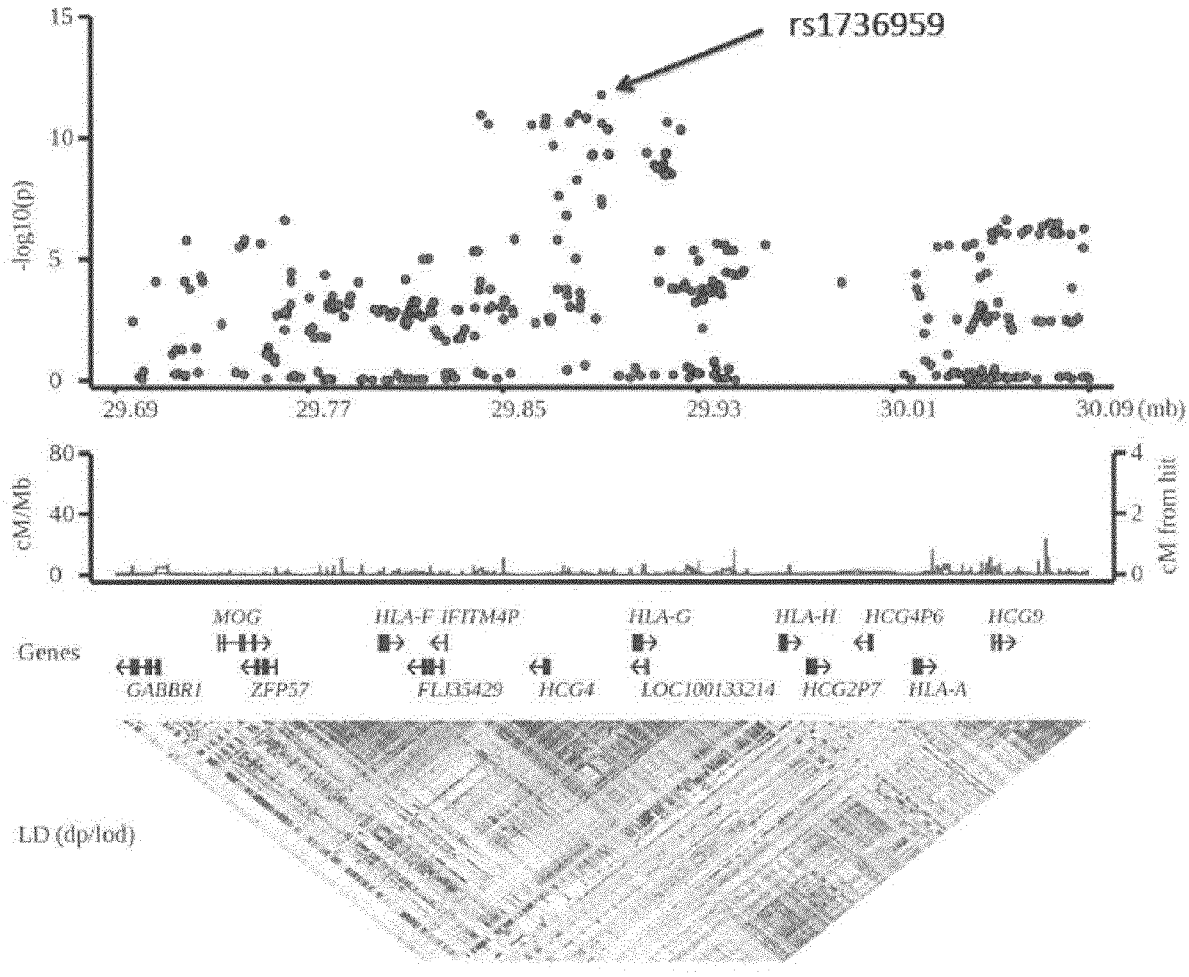
rs1736959
15
10
5
0
-log10(p)
29.69
29.77
29.85
29.93
30.01
30.09 (mb)
80
40
0
cM/Mb
4
2
0
cM from hit
Genes
MOG
HLA-F IFITM4P
HLA-G
HLA-H
HCG4P6
HCG9
GABBR1
ZFP57
FLJ35429
HCG4
LOC100133214
HCG2P7
HLA-A
LD (dp/lod)

Fig. 6

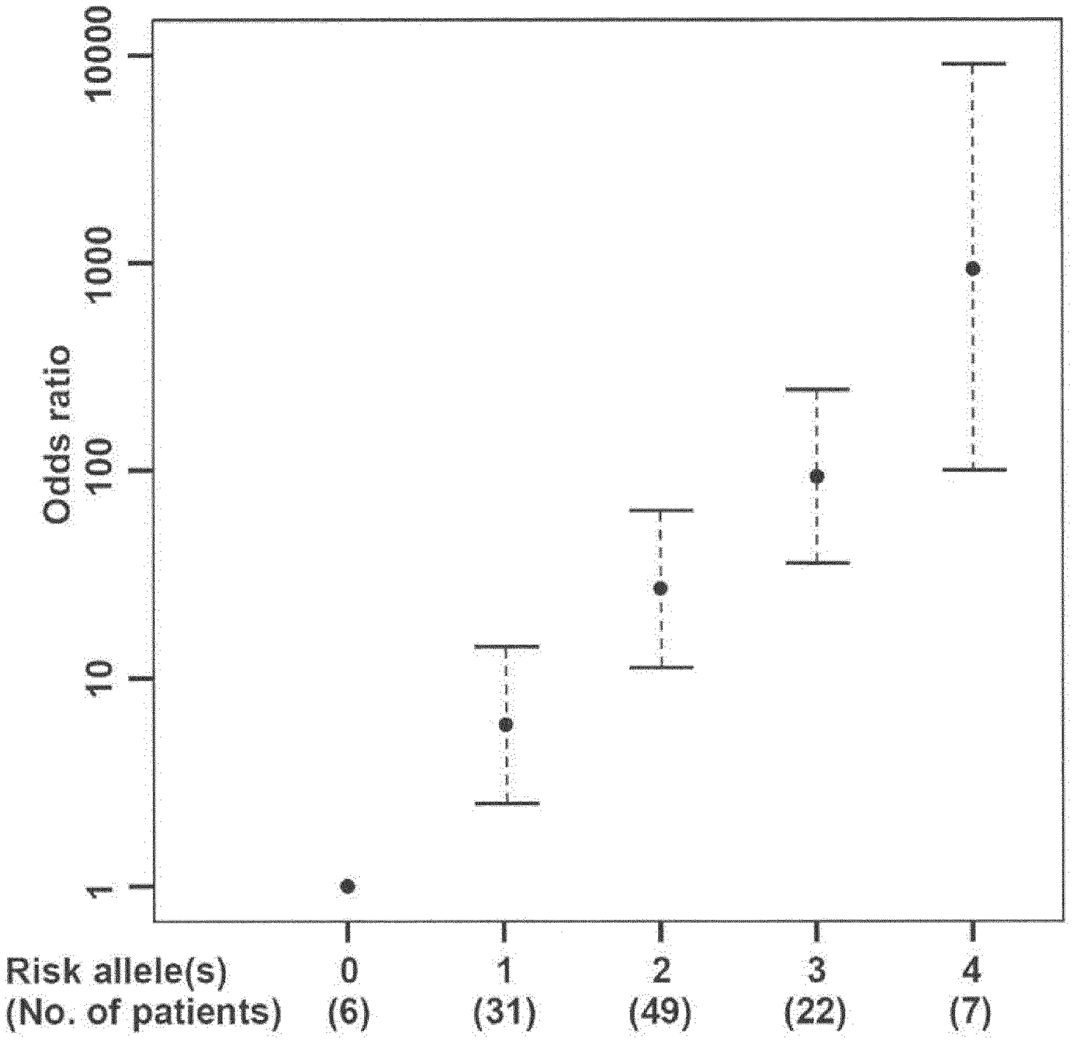
Odds ratio
10000
1000
100
10
1
Risk allele(s)
(No. of patients)
0
(6)
1
(31)
2
(49)
3
(22)
4
(7)

Fig. 7

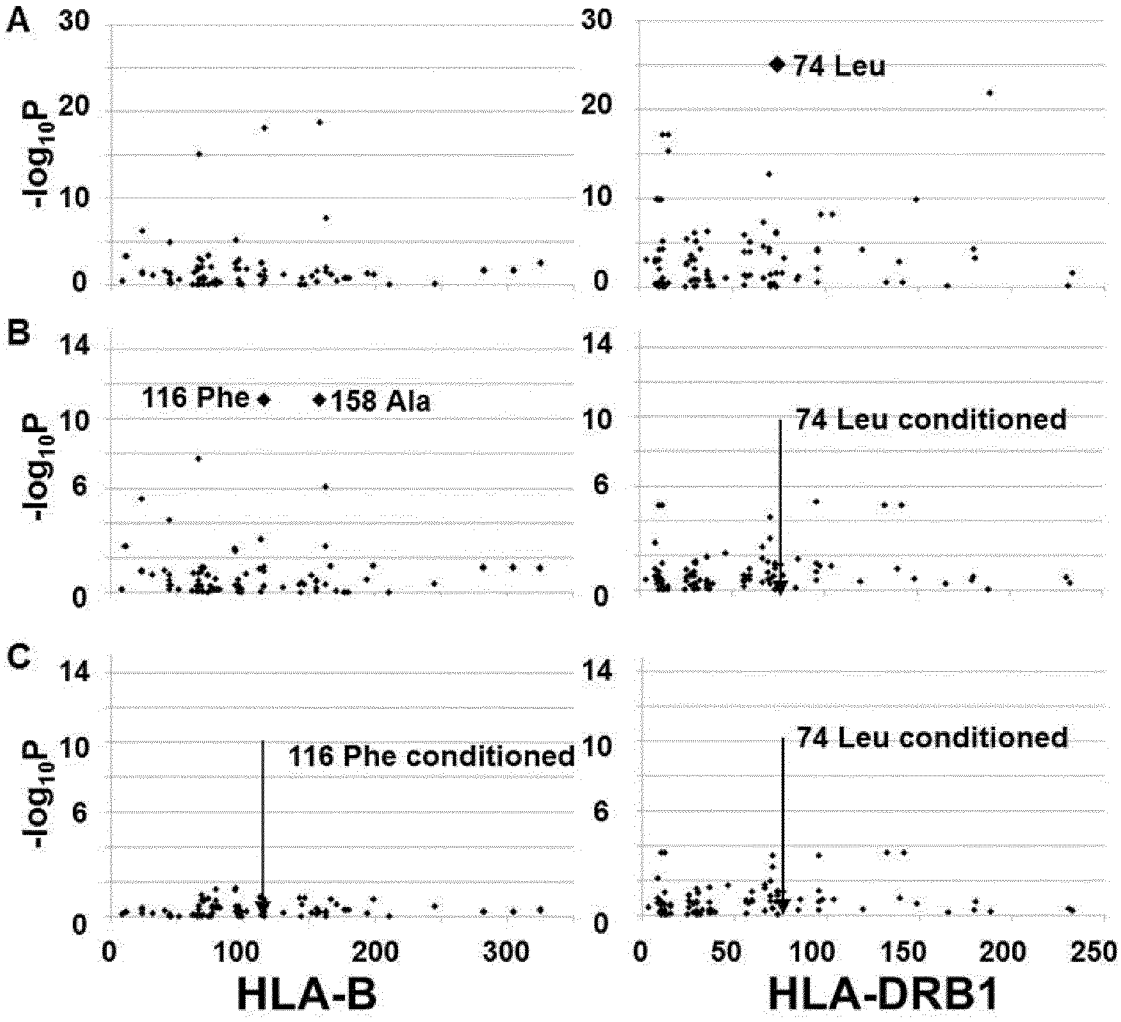
A
B
C
-log10P
30
20
10
0
14
10
6
74 Leu
116 Phe
158 Ala
74 Leu conditioned
116 Phe conditioned
0
100
200
300
50
150
250
HLA-B
HLA-DRB1

## INTERNATIONAL SEARCH REPORT

International application No.
PCT/JP2014/074145

A. CLASSIFICATION OF SUBJECT MATTER
*C12Q1/68*(2006.01)i, *C12N15/09*(2006.01)i

According to International Patent Classification (IPC) or to both national classification and IPC

B. FIELDS SEARCHED

Minimum documentation searched (classification system followed by classification symbols)
C12Q1/68, C12N15/09

Documentation searched other than minimum documentation to the extent that such documents are included in the fields searched

Electronic data base consulted during the international search (name of data base and, where practicable, search terms used)
CAplus/MEDLINE/EMBASE/BIOSIS(STN), WPIDS/WPIX(STN), JSTPlus/JMEDPlus/JST7580(JDreamIII), GeneCards

C. DOCUMENTS CONSIDERED TO BE RELEVANT

| Category* | Citation of document, with indication, where appropriate, of the relevant passages | Relevant to claim No. |
|---|---|---|
| A | TAMAI, H., et al., Association between the DRB1*08032 histocompatibility antigen and methimazole-induced agranulocytosis in Japanese patients with Graves disease, Ann. Intern. Med., 1996, vol.124, no.5, p.490-494 | 1-13 |
| A | US 5223399 A (LONG ISLAND JEWISH MEDICAL CENTER), 29 June 1993 (29.06.1993), (Family: none) | 1-13 |
| A | US 2006/0177860 A1 (Maria ATHANASIOU et al.), 10 August 2006 (10.08.2006), (Family: none) | 1-13 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 03 December, 2014 (03.12.14) | 16 December, 2014 (16.12.14) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2014/074145

C (Continuation). DOCUMENTS CONSIDERED TO BE RELEVANT

| Category* | Citation of document, with indication, where appropriate, of the relevant passages | Relevant to claim No. |
|---|---|---|
| A | US 2006/0183146 A1 (Maria ATHANASIOU et al.), 17 August 2006 (17.08.2006), (Family: none) | 1-13 |
| A | AMAR, A., et al., An association between clozapine-induced agranulocytosis in schizophrenics and HLA-DQB1*0201, Int. J. Neuropsychopharmacol., 1998, vol.1, no.1, p.41-44 | 1-13 |
| A | ATHANASIOU, MC., et al., Candidate gene analysis identifies a polymorphism in HLA-DQB1 associated with clozapine-induced agranulocytosis, J. Clin. Psychiatry, 2011, vol.72, no.4, p.458-463 | 1-13 |
| A | JP 2005-058224 A (Otsuka Pharmaceutical Co., Ltd.), 10 March 2005 (10.03.2005), & WO 2005/010183 A1 & EP 1650300 A1 & US 2007/0264631 A1 | 1-13 |
| A | VLAHOV, V., et al., Genetic factors and risk of agranulocytosis from metamizol, Pharmacogenetics, 1996, vol.6, no.1, p.67-72 | 1-13 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 03023063 A
- JP 2013188806 A


### Non-patent literature cited in the description


- **TAMAI H et al.** *Ann Intern Med.,* 1996, vol. 124 (5), 490-494
- **WALLACE et al.** *Proc. Natl. Acad. Sci. USA,* 1983, vol. 80, 278-282
- **ANDERSSON et al.** *European journal of epidemiology,* 2005, vol. 20, 575-9